# EUROPEAN PATENT APPLICATION

(11) **EP 4 785 979 A2**
(43) Date of publication of application: **05.08.2026**
(21) Application number: 26185282.6
(22) Date of filing: 22.12.2021
(51) Int. Cl.: A61M 16/16

(54) **TUBE AND/OR PATIENT INTERFACE FOR DELIVERY OF GAS**

(30) Priority: 23.12.2020 US 202063130313 P
(62) Divisional of application: 21909679.9
(71) Applicant: Fisher & Paykel Healthcare Limited, East Tamaki, Auckland 2013 (NZ)
(72) Inventor: GADSBY, Georgia, 2013 Auckland (NZ); STANLEY, Leon Tyler, 2013 Auckland (NZ); RONAYNE, Michael Paul, 2013 Auckland (NZ); HILLIARD, Andrew James, 2013 Auckland (NZ); MONRO, Rory Alexander, 2013 Auckland (NZ)
(74) Representative: Forresters IP LLP

(57) **Abstract**

A patient interface for delivery of gas to a patient. The patient interface has at least one inspiratory lumen for directing a flow of gas to a patient airway; at least one expiratory lumen comprising an expiratory gas flow path for directing a flow of expiratory gases from the patient; and a diffuser in fluid communication with, or part of, the expiratory gas flow path, the diffuser configured to diffuse the flow of expiratory gases from the patient interface before passage of the flow of expiratory gases from the patient interface into ambient air.

## Description

### TECHNICAL FIELD

The present disclosure relates to a tube for a patient interface for delivery of gas to a patient, and to an associated patient interface. It relates particularly, but not exclusively, to a tube or patient interface for directing or treating expiratory gases from the patient.

### BACKGROUND

In various medical procedures respiratory support is required to achieve adequate gas exchange in the patient. Respiratory support involves delivery of respiratory gas to the patient's airway via a patient interface such as a nasal cannula, endotracheal tube (ETT), tracheostomy tube, laryngeal mask (LMA), nasal mask, face mask or the like. Respiratory support may be provided in an apnoeic patient, or in a patient who is spontaneously breathing. In many cases, it is desirable to provide respiratory gas to the patient at high flow rates to achieve effective oxygenation and to clear CO2, reducing respiratory dead space.

Patients receiving respiratory support are typically very unwell and delivery of respiratory support is a significant factor in prospects and rates of recovery. Therefore, advances in respiratory support delivery systems and patient interfaces have focussed on features for safe and effective delivery of respiratory gas to the patient with little attention paid to management of expiratory gases.

The discussion of the background included herein including reference to documents, acts, materials, devices, articles and the like is included to explain the context of the present disclosure. This is not to be taken as an admission or a suggestion that any of the material referred to was published, known or part of the common general knowledge in any jurisdiction as at the priority date of any of the claims.

### SUMMARY

Numerous respiratory illnesses are known to be transmitted through infected droplets of respiratory gases entering the respiratory system of uninfected subjects. The 2009 "Swine flu" A(H1N1) began in Mexico and rapidly spread to over 200 countries and territories. Coronaviruses form a large family of viruses that can cause a range of illnesses from the common cold to more serious diseases. SARS (severe acute respiratory syndrome), MERS (Middle East respiratory syndrome) and the more recent respiratory syndrome coronavirus 2 (SARS-CoV-2) which is responsible for causing the disease COVID-19 have reached pandemic status due to their high rates of transmission. According to the World Health Organisation (WHO) as at the priority date of this application, the transmission of severe acute SARS-CoV-2 is thought to occur primarily via droplets and fomites between an infector and infectee. Subsequent research and evidence now suggests that infection can occur when aerosols or droplets containing virus are inhaled or come directly into contact with the eyes, nose, or mouth.

Aerosols, being a suspension of tiny particles or droplets in the air, can be generated through various mechanisms, including the interaction of a high velocity air stream with that of a relatively slow-moving flow or source of liquid. People routinely produce respiratory particles in a range of sizes that include droplets, aerosols, and particles in between from activities such as speaking, breathing, exercise, sneezing and coughing. These tiny particles or droplets can contain substances and/or contaminants that are undesirable for persons to come in contact with. The aerosols may be infective and/or contain pathogens and, if inhaled or contacted with fluids from the eye, may cause adverse health effects.

Management of expiratory gases has at least a perceived potential to become particularly challenging during delivery of high flow therapy with a non-sealing patient interface.

The risk of transmission of infection depends on the quantity of viable pathogen that makes its way to a site of infection in the infectee, which in turn depends on the quantity of pathogen emitted through respiratory or otherwise expelled gases and the survival rate of the pathogen during transmission. Similarly, the risk of exposure to substances or contaminants, such as nebulized drugs, in a patient's expiratory gases depends on the quantity of drug that makes its way to an unintended recipient, which in turn depends on the quantity of drug emitted through respiratory gases, and the viability of the drug in the unintended recipient.

It would be desirable to mitigate one or more risks associated with expiratory gases from patients receiving respiratory support.

In a first aspect, in accordance with certain features, aspects and advantages of at least one of the embodiments disclosed herein, a tube for delivery of gases to a patient interface and for treatment of expiratory gases is disclosed, the tube comprising:
an inspiratory conduit for directing a flow of gas toward a patient interface;
an expiratory conduit for directing a flow of expiratory gases from the patient interface;
and a filter in fluid communication with, or part of, the expiratory conduit, the filter configured to filter the flow of expiratory gases from the patent interface before passage of the flow of expiratory gases from the patient interface into ambient air.

In some configurations, the inspiratory conduit and the expiratory conduit are coaxial or substantially parallel.

In some configurations, the inspiratory conduit and the expiratory conduit are on a single limb, such that the inspiratory conduit and the expiratory conduit both extend in one direction from the patient interface in use.

In some configurations, the inspiratory conduit is an inner conduit, and wherein the expiratory conduit is an outer conduit.

In some configurations, a wall of the expiratory conduit comprises the filter.

In some configurations, the filter comprises one or more filter materials selected from the group comprising: an N95 or higher (e.g. N99) rated or HEPA filter material, fibrous, porous knitted, mesh, woven foam, textile, non-textile, or foam filter material.

In some configurations, a length of the filter in the wall is configured to reduce a resistance to flow of gases through the filter.

In some configurations, a portion of the expiratory conduit upstream of the filter has a wall that is substantially non-permeable.

In some configurations, the portion of the expiratory conduit upstream of the filter has a length of at least about 80 mm.

In some configurations, the expiratory conduit is coupled to a filter component that comprises the filter.

In some configurations, the filter comprises one or more filter materials selected from the group comprising: an N95 or higher (e.g. N99) rated or HEPA filter material, fibrous, porous knitted, mesh, woven foam, textile, non-textile, or foam filter material.

In some configurations, the expiratory conduit comprises structure to prevent or minimise collapse or constriction of an expiratory lumen in the expiratory conduit.

In some configurations, the structure comprises one more of: corrugations, pleats, helical wrap, ribs, supporting features.

In some configurations, the inspiratory conduit is configured to direct a flow of gas toward at least one inspiratory lumen of the patient interface, and wherein the expiratory conduit is configured to direct a flow of gas from at least one expiratory lumen of the patient interface.

In a further aspect, in accordance with certain features, aspects and advantages of at least one of the embodiments disclosed herein, the combination of a patient interface and the tube as outlined herein is disclosed, wherein the patient interface comprises: at least one inspiratory element for directing a flow of gas to a patient airway, the or each inspiratory element comprising the at least one inspiratory lumen through which said flow of gas is directed; and at least one expiratory element comprising the expiratory lumen that provides an expiratory gas flow path.

In some configurations, the patient interface comprises first and second nasal elements configured to direct gas into and/or from a nare of the patient.

In some configurations, the at least one inspiratory element comprises one or both of the first and second nasal elements, and wherein the at least one expiratory element comprises one or both of the first and second nasal elements.

In some configurations, the first nasal element comprises a first nasal prong and wherein the second nasal element comprises a second nasal prong.

In some configurations, the patient interface comprises a flow directing member to direct gas flow from the inspiratory conduit to one of the first and second nasal elements, and to direct gas flow from the other of the first and second nasal elements to the expiratory conduit.

In some configurations, the patient interface comprises a partition or other physical separation to provide for a mechanical fluid disconnection between a fluid flow path of the first nasal element and a fluid flow path of the second nasal element.

In some configurations, the patient interface comprises a gases port for entrainment of expiratory gases from a patient's nose and/or a patient's mouth that are not directed into an expiratory nasal element.

In a further aspect, in accordance with certain features, aspects and advantages of at least one of the embodiments disclosed herein, an expiratory tube for treatment of expiratory gases is disclosed, the expiratory tube comprising:
an expiratory conduit for directing a flow of expiratory gases from a patient interface;
and a filter in fluid communication with, or part of, the expiratory conduit, the filter configured to filter the flow of expiratory gases from the patent interface before passage of the flow of expiratory gases from the patient interface into ambient air.

In some configurations, a wall of the expiratory conduit comprises the filter.

In some configurations, the filter comprises one or more filter materials selected from the group comprising: an N95 or higher (e.g. N99) rated or HEPA filter material, fibrous, porous knitted, mesh, woven foam, textile, non-textile, or foam filter material.

In some configurations, a length of the filter in the wall is configured to reduce a resistance to flow of gases through the filter.

In some configurations, a portion of the expiratory conduit upstream of the filter has a wall that is substantially non-permeable.

In some configurations, the portion of the expiratory conduit upstream of the filter has a length of at least about 80 mm.

In some configurations, the expiratory conduit is coupled to a filter component that comprises the filter.

In some configurations, the filter comprises one or more filter materials selected from the group comprising: an N95 or higher (e.g. N99) rated or HEPA filter material, fibrous, porous knitted, mesh, woven foam, textile, non-textile, or foam filter material.

In some configurations, the expiratory conduit comprises structure to prevent or minimise collapse or constriction of an expiratory lumen in the expiratory conduit.

In some configurations, the structure comprises one more of: corrugations, pleats, helical wrap, ribs, supporting features.

In some configurations, the expiratory conduit is configured to direct a flow of gas from at least one expiratory lumen of the patient interface.

In a further aspect, in accordance with certain features, aspects and advantages of at least one of the embodiments disclosed herein, the combination of a patient interface and the expiratory tube as outlined herein is disclosed, wherein the patient interface comprises: at least one inspiratory element for directing a flow of gas to a patient airway, the or each inspiratory element comprising the at least one inspiratory lumen through which said flow of gas is directed; and at least one expiratory element comprising the expiratory lumen that provides an expiratory gas flow path.

In some configurations, the patient interface comprises first and second nasal elements configured to direct gas into and/or from a nare of the patient.

In some configurations, the at least one inspiratory element comprises one or both of the first and second nasal elements, and wherein the at least one expiratory element comprises one or both of the first and second nasal elements.

In some configurations, the first nasal element comprises a first nasal prong and wherein the second nasal element comprises a second nasal prong.

In a further aspect, in accordance with certain features, aspects and advantages of at least one of the embodiments disclosed herein, a patient interface for delivery of gas to a patient is disclosed, the patient interface comprising:
at least one inspiratory lumen for directing a flow of gas to a patient airway;
at least one expiratory lumen comprising an expiratory gas flow path for directing a flow of expiratory gases from the patient;
   and
a filter in fluid communication with, or part of, the expiratory gas flow path, the filter configured to filter the flow of expiratory gases from the patent interface before passage of the flow of expiratory gases from the patient interface into ambient air.

In some configurations, the patient interface comprises an inspiratory conduit that is in fluid communication with the inspiratory lumen, and an expiratory conduit that is in fluid communication with the expiratory lumen.

In some configurations, the inspiratory conduit and the expiratory conduit are integrally formed with the patient interface or are coupled to the patient interface.

In some configurations, the inspiratory conduit and the expiratory conduit are coaxial or substantially parallel.

In some configurations, the inspiratory conduit and the expiratory conduit are on a single limb, such that the inspiratory conduit and the expiratory conduit both extend in one direction from the patient interface.

In some configurations, the inspiratory conduit is an inner conduit, and wherein the expiratory conduit is an outer conduit.

In some configurations, a wall of the expiratory conduit comprises the filter.

In some configurations, the filter comprises one or more filter materials selected from the group comprising: an N95 or higher (e.g. N99) rated or HEPA filter material, fibrous, porous knitted, mesh, woven foam, textile, non-textile, or foam filter material.

In some configurations, a length of the filter in the wall is configured to reduce a resistance to flow of gases through the filter.

In some configurations, a portion of the expiratory conduit upstream of the filter has a wall that is substantially non-permeable.

In some configurations, the portion of the expiratory conduit upstream of the filter has a length of at least about 80 mm.

In some configurations, the expiratory conduit is coupled to a filter component that comprises the filter.

In some configurations, the filter comprises one or more filter materials selected from the group comprising: an N95 or higher (e.g. N99) rated or HEPA filter material, fibrous, porous knitted, mesh, woven foam, textile, non-textile, or foam filter material.

In some configurations, the expiratory conduit comprises structure to prevent or minimise collapse or constriction of an expiratory lumen in the expiratory conduit.

In some configurations, the structure comprises one more of: corrugations, pleats, helical wrap, ribs, supporting features.

In some configurations, the patient interface comprises first and second nasal elements configured to direct gas into and/or from a nare of the patient.

In some configurations, the first nasal element comprises a first nasal prong, and wherein the second nasal element comprises a second nasal prong.

In some configurations, the patient interface comprises a flow directing member to direct gas flow to one of the first and second nasal elements, and to direct gas flow from the other of the first and second nasal elements.

In some configurations, the patient interface comprises a partition or other physical separation to provide for a mechanical fluid disconnection between a fluid flow path of the first nasal element and a fluid flow path of the second nasal element.

In some configurations, the patient interface comprises a gases port for entrainment of expiratory gases from a patient's nose and/or a patient's mouth that are not directed into an expiratory nasal element.

In a further aspect, in accordance with certain features, aspects and advantages of at least one of the embodiments disclosed herein, a patient interface for respiratory therapy is disclosed, the patient interface comprising:
a cannula body comprising a first nasal element and a second nasal element; and
a cannula manifold, the cannula manifold comprising a manifold inspiratory lumen, a manifold expiratory lumen, and a flow directing member, the cannula manifold being reconfigurable relative to the cannula body such that in a first configuration the flow directing member is arranged to direct gas flow from the manifold inspiratory lumen to the first nasal element and to direct gas flow from the second nasal element to the manifold expiratory lumen, and in a second configuration the flow directing member is arranged to direct gas flow from the manifold inspiratory lumen to the second nasal element and to direct gas flow from the first nasal element to the manifold expiratory lumen.

In some configurations, the cannula manifold is configured for insertion into the cannula body.

In some configurations, the patient interface comprises or is coupled to an inspiratory conduit that is in fluid communication with the inspiratory lumen, and comprising or coupled to an expiratory conduit that in fluid communication with the expiratory lumen.

In some configurations, the inspiratory conduit and the expiratory conduit are coaxial or substantially parallel.

In some configurations, the inspiratory conduit and the expiratory conduit are on a single limb, such that the inspiratory element and the expiratory element both extend in one direction from the cannula body.

In some configurations, the inspiratory conduit is an inner conduit, and the expiratory conduit is an outer conduit.

In some configurations, the flow directing member is arranged such that a distal one of the first and second nasal elements relative to a manifold inlet and a manifold outlet is an inspiratory nasal element for directing gas flow to the patient and a proximal one of the first and second nasal elements relative to the manifold inlet and the manifold outlet is an expiratory nasal element for directing a flow of expiratory gases away from the patient.

In some configurations, the flow directing member is arranged such that a proximal one of the first and second nasal elements relative to a manifold inlet and a manifold outlet is an inspiratory nasal element for directing gas flow to the patient and a distal one of the first and second nasal elements relative to the manifold inlet and the manifold outlet is an expiratory nasal element for directing a flow of expiratory gases away from the patient.

In some configurations, the patient interface comprises a gases port for entrainment of expiratory gases from a patient's nose and/or a patient's mouth that are not directed into the expiratory nasal element.

In some configurations, the cannula manifold comprises a body portion, and wherein the flow directing member comprises a proximal portion that extends substantially in a longitudinal direction of a body portion and a distal portion that extends transversely from a distal end of the proximal portion to meet the body portion.

In some configurations, a cavity defined between the proximal portion, the distal portion, and the body portion defines a cavity to form one of an expiratory gases receipt opening and an inspiratory gas delivery opening.

In some configurations, the other of the inspiratory gas delivery opening and the expiratory gases receipt opening is provided on an opposite side of the distal portion of the flow directing member from the cavity.

In some configurations, the first and second nasal elements are configured to direct gas into and/or from a nare of the patient.

In some configurations, the first nasal element comprises a first nasal prong, and the second nasal element comprises a second nasal prong.

In some configurations, the cannula manifold is configured to be inserted into the cannula body in the first configuration and in the second configuration.

In some configurations, the first configuration corresponds to the cannula manifold being inserted into the cannula body from a first side of the cannula body, and the second configuration corresponds to the cannula manifold being inserted into the cannula body from a second side of the cannula body.

In some configurations, the cannula body comprises an elastomeric component.

In some configurations, the cannula manifold comprises a rigid polymer component.

In some configurations, the cannula manifold comprises a pressure monitoring port, wherein the pressure monitoring port is in fluid communication with the manifold inspiratory lumen and the manifold expiratory lumen.

In some configurations, the pressure monitoring port is configured to provide a gas flow for pressure monitoring through the pressure monitoring port, the gas flow comprising an average of the inspiratory gas flow and expiratory gas flow.

In some configurations, the patient interface comprises a filter in fluid communication with the manifold expiratory lumen, the filter configured to filter the flow of gas from the patent interface before passage of the flow of expiratory gases from the patient interface into ambient air.

In a further aspect, in accordance with certain features, aspects and advantages of at least one of the embodiments disclosed herein, a patient interface for respiratory therapy is disclosed, the patient interface comprising:
a cannula body comprising a first nasal element configured to direct gas into a nare of the patient and a second nasal element configured to direct gas from another nare of the patient; and
a cannula manifold, the cannula manifold comprising a manifold inspiratory lumen for inspiratory gas flow in fluid communication with a manifold inlet, a manifold expiratory lumen for expiratory gas flow in fluid communication with a manifold outlet, a flow directing member that is arranged to direct gas flow from the manifold inspiratory lumen to the first nasal element and to direct gas flow from the second nasal element to the manifold expiratory lumen, and a pressure monitoring port in fluid communication with the manifold inspiratory lumen and the manifold expiratory lumen, wherein the manifold inlet and the manifold outlet are on a single limb, such that the manifold inlet and the manifold outlet both extend in one direction from the patient interface in use.

In some configurations, the patient interface comprises a lumen in fluid communication with the pressure monitoring port, an inspiratory gases port in fluid communication with the manifold inspiratory lumen, and an expiratory gases port in fluid communication with the manifold expiratory lumen, wherein the lumen provides fluid communication between the inspiratory gases port, the expiratory gases port, and the pressure monitoring port.

In some configurations, the manifold inlet and the manifold outlet are coaxial or substantially parallel.

In some configurations, the patient interface comprises an inspiratory conduit that is in fluid communication with the manifold inspiratory lumen, and an expiratory conduit that is in fluid communication with the manifold expiratory lumen.

In some configurations, the inspiratory conduit and the expiratory conduit are integrally formed with the cannula manifold or are coupled to the cannula manifold.

In some configurations, the pressure monitoring port is configured to provide a gas flow for pressure monitoring through the pressure monitoring port, the gas flow comprising an average of the inspiratory gas flow and expiratory gas flow.

In some configurations, the cannula manifold is configured for insertion into the cannula body.

In some configurations, the cannula manifold is configured to be inserted into the cannula body in a first and configuration and in a second configuration, and wherein the first configuration corresponds to the cannula manifold being inserted into the cannula body from a first side of the cannula body, and wherein the second configuration corresponds to the cannula manifold being inserted into the cannula body from a second side of the cannula body.

In a further aspect, in accordance with certain features, aspects and advantages of at least one of the embodiments disclosed herein, a patient interface for respiratory therapy is disclosed, the patient interface comprising:
a cannula body comprising a first nasal element and a second nasal element; and
a cannula manifold, the cannula manifold comprising a manifold inspiratory lumen, a manifold expiratory lumen, and a flow directing member, wherein the flow directing member is arranged to direct gas flow from the manifold inspiratory lumen to the first nasal element and to direct gas flow from the second nasal element to the manifold expiratory lumen.

In some configurations, the cannula manifold is configured for insertion into the cannula body.

In some configurations, the patient interface comprises or is coupled to an inspiratory conduit that is in fluid communication with the inspiratory lumen, and comprising or coupled to an expiratory conduit that in fluid communication with the expiratory lumen.

In some configurations, the inspiratory conduit and the expiratory conduit are coaxial or substantially parallel.

In some configurations, the inspiratory conduit and the expiratory conduit are on a single limb, such that the inspiratory element and the expiratory element both extend in one direction from the cannula body.

In some configurations, the inspiratory conduit is an inner conduit, and wherein the expiratory conduit is an outer conduit.

In some configurations, the flow directing member is arranged such that a distal one of the first and second nasal elements relative to a manifold inlet and a manifold outlet is an inspiratory nasal element for directing gas flow to the patient and a proximal one of the first and second nasal elements relative to the manifold inlet and the manifold outlet is an expiratory nasal element for directing a flow of expiratory gases away from the patient.

In some configurations, the flow directing member is arranged such that a proximal one of the first and second nasal elements relative to a manifold inlet and a manifold outlet is an inspiratory nasal element for directing gas flow to the patient and a distal one of the first and second nasal elements relative to the manifold inlet and the manifold outlet is an expiratory nasal element for directing a flow of expiratory gases away from the patient.

In some configurations, the patient interface comprises a gases port for entrainment of expiratory gases from a patient's nose and/or a patient's mouth that are not directed into an expiratory nasal element.

In some configurations, the cannula manifold comprises a body portion, and wherein the flow directing member comprises a proximal portion that extends substantially in a longitudinal direction of a body portion and a distal portion that extends transversely from a distal end of the proximal portion to meet the body portion.

In some configurations, a cavity defined between the proximal portion, the distal portion, and the body portion defines a cavity to form one of an expiratory gases receipt opening and an inspiratory gas delivery opening.

In some configurations, the other of the inspiratory gas delivery opening and the expiratory gases receipt opening is provided on an opposite side of the distal portion of the flow directing member from the cavity.

In some configurations, the first and second nasal elements are configured to direct gas into and/or from a nare of the patient.

In some configurations, the first nasal element comprises a first nasal prong, and wherein the second nasal element comprises a second nasal prong.

In some configurations, the cannula manifold is configured to be inserted into the cannula body.

In some configurations, the cannula body comprises an elastomeric component.

In some configurations, the cannula manifold comprises a rigid polymer component.

In some configurations, the cannula manifold comprises a pressure monitoring port, wherein the pressure monitoring port is in fluid communication with the manifold inspiratory lumen and the manifold expiratory lumen.

In some configurations, the pressure monitoring port is configured to provide a gas flow for pressure monitoring through the pressure monitoring port, the gas flow comprising an average of the inspiratory gas flow and expiratory gas flow.

In some configurations, the patient interface comprises a filter in fluid communication with the manifold expiratory lumen, the filter configured to filter the flow of gas from the patent interface before passage of the flow of expiratory gases from the patient interface into ambient air.

In a further aspect, in accordance with certain features, aspects and advantages of at least one of the embodiments disclosed herein, a respiratory support or assistance system is disclosed, the respiratory support or assistance system comprising: the patient interface as outlined herein and at least one of a humidifier and a respiratory gas source.

In some configurations, the respiratory support or assistance system comprises an inspiratory conduit for delivering gases from the humidifier or respiratory gas source to the patient interface.

In a further aspect, in accordance with certain features, aspects and advantages of at least one of the embodiments disclosed herein, a kit is disclosed, the kit comprising: the tube as outlined herein or the expiratory tube as outlined herein and a patient interface.

In some configurations, the patient interface is as outlined herein.

It is to be noted that any one of the aspects mentioned above may include any of the features of any of the other aspects and may include any of the features of any of the embodiments in the following sections.

It is to be understood each of the various aspects described herein may incorporate features, modifications and alternatives described in the context of one or more other aspects, such as but not limited to the various features of aspects of the tube, filter, and patient interface, which may incorporate any one or more of the various features of the tube, filter, and patient interface as described in the context of the various aspects of the patient interface. For efficiency, such features, modifications and alternatives have not been repetitiously disclosed for each and every aspect although one of skill in the art will appreciate that such combinations of features, modifications and alternatives disclosed for some aspects apply similarly for other aspects and are within the scope of and form part of the subject matter of this disclosure.

As used herein the term "(s)" following a noun means the plural and/or singular form of that noun.

As used herein the term "and/or" means "and" or "or", or where the context allows both.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

This disclosure may also be said broadly to consist in the parts, elements and features referred to or indicated in the specification of the application, individually or collectively, and any or all combinations of any two or more said parts, elements or features, and where specific integers are mentioned herein which have known equivalents in the art to which this disclosure relates, such known equivalents are deemed to be incorporated herein as if individually set forth.

The disclosure consists in the foregoing and also envisages constructions of which the following gives examples only.

### BRIEF DESCRIPTION OF DRAWINGS

Specific embodiments and modifications thereof will become apparent to those skilled in the art from the detailed description herein having reference to the figures that follow, of which:
Figure 1 is a schematic view of an overall respiratory assistance or support system.
Figure 2 is a schematic illustration of a first configuration patient interface having nasal elements and with filtration provided by a filter remotely located from the nasal elements.
Figure 3 shows schematic cross-sections of exemplary configurations of inspiratory and expiratory conduits for us with the patient interfaces described herein or other uses, where Figure 3(a) shows a co-axial configuration where the inspiratory conduit is inside the expiratory conduit, Figure 3(b) shows a separate side-by-side inspiratory conduit and expiratory conduit which may be parallel, and Figure 3(c) shows a conjoined inspiratory conduit and expiratory conduit.
Figure 4A is a schematic illustration of a second configuration patient interface and tube.
Figure 4B is a schematic illustration of a third configuration patient interface and tube.
Figure 4C is a schematic illustration of a fourth configuration patient interface and tube.
Figure 4D is a schematic illustration of a fifth configuration patient interface and tube.
Figure 4E is a schematic illustration of a sixth configuration patient interface and tube.
Figure 4F is a schematic illustration of a seventh configuration patient interface and tube.
Figure 4G is a schematic illustration of an eighth configuration patient interface and tube.
Figure 4H is a schematic illustration of a ninth configuration patient interface and tube.
Figure 5A is a front view of a patient interface showing alternative insertion directions of a cannula manifold into the cannula body of a patient interface.
Figure 5B is a front overhead perspective view of the patient interface of Figure 4A.
Figure 5C is an overhead view of the patient interface of Figure 4A.
Figure 5D is an underside perspective view of the body of the patient interface with the manifold not shown.
Figure 5E is a front sectional view of the body of Figure 5D.
Figure 5F is an overhead view of the cannula manifold of the patient interface of Figure 5A.
Figure 5G is a left side sectional view of the cannula manifold of Figure 5F.
Figure 5H is an overhead view of the cannula manifold of Figure 5F.
Figure 5I is a front overhead perspective view of the cannula manifold of Figure 5F.
Figure 5J is a front sectional view of the patient interface and cannula manifold assembly.
Figure 5K is an overhead sectional view of the patient interface and cannula manifold.
Figure 5L is a front overhead sectional perspective view of the patient interface and cannula manifold.
Figure 5M is another front overhead sectional perspective view of the patient interface and cannula manifold.
Figure 5N is a rear overhead perspective view of the patient interface and cannula manifold with the overmould section not shown.
Figure 5O is another rear overhead perspective view of the patient interface and cannula manifold with the overmould section not shown.
Figure 6A is a schematic view of the gas flow paths in a first configuration of the patient interface and cannula manifold.
Figure 6B is a schematic view of the gas flow paths in a second configuration of the patient interface and cannula manifold.
Figure 6C is a schematic view of the gas flow paths in a first configuration of the patient interface and an alternative cannula manifold.
Figure 6D is a schematic view of the gas flow paths in a second configuration of the patient interface and the alternative cannula manifold.
Figure 7A is an overhead view of the alternative cannula manifold for use with the patient interface.
Figure 7B is a front view of the alternative cannula manifold.
Figure 7C is an underside view of the alternative cannula manifold.
Figure 7D is a left side sectional view of the alternative cannula manifold.
Figure 7E is a sectional front overhead perspective view of the alternative cannula manifold with inspiratory gases flow represented by solid lines with arrows and expiratory gases flow represented by dot-dash lines with arrows.
Figure 8A is an overhead view of an alternative cannula manifold with a pressure monitoring port.
Figure 8B is a front view of the alternative cannula manifold.
Figure 8C is an underside view of the alternative cannula manifold.
Figure 8D is a rear view of the alternative cannula manifold.
Figure 8E is a front overhead perspective view of the alternative cannula manifold.
Figure 8F is a left side sectional view of the alternative cannula manifold showing details of the pressure monitoring port.
Figure 8G is another left side sectional view of the alternative cannula manifold showing details of the pressure monitoring port.
Figure 9A is an overhead view of another alternative cannula manifold with a pressure monitoring port and a pressure tube.
Figure 9B is a front view of the alternative cannula manifold.
Figure 9C is an underside view of the alternative cannula manifold.
Figure 9D is a front overhead perspective view of the alternative cannula manifold.
Figure 9E is a rear overhead perspective view of the alternative cannula manifold showing the pressure monitoring port.
Figure 9F is a sectional view of the alternative cannula manifold showing details of the pressure monitoring port.
Figure 10A shows a patient interface in use on a patient, the patient interface having an external flow director on the cannula body for capturing and redirecting expiratory flow.
Figure 10B is a front perspective view of the external flow director.
Figure 10C shows a front cross-sectional view of the external flow director.
Figure 10D shows an alternative configuration of the external flow director.
Figure 10E shows an alternative configuration of the external flow director.

It is to be understood that the embodiments shown are examples only and are not to be taken as limiting the scope of the disclosure.

### DETAILED DESCRIPTION

Embodiments of the present disclosure are directed to a patient interface used in respiratory support which provides features for directing or treating expiratory gases from the patient. Treatment of expiratory gases may reduce transmission risk in circumstances where those gases may contain infective or pathogenic particles, as well as reducing risks associated with presence of other substances, for example, nebulised drugs, in expiratory gases. Some embodiments of the disclosure are particularly directed to reducing transmission risk arising from aerosols in the expiratory gases during the provision of respiratory therapy, although it is to be understood that the scope of this disclosure is not limited to such application. The present disclosure provides embodiments for intercepting/capturing and/or slowing the velocity of aerosols and other particles in expiratory gases to reduce the concentration of particles in the flow of expiratory gases to reduce the likelihood of those particles travelling a sufficient distance to either enter the airway of another person or land on a surface that could present a transfer risk when touched.

One factor in determining if respiratory droplets pose a risk of becoming airborne is the size of the droplets. Respiratory droplets come in a range of sizes, characterized by diameters with an upper limit of several hundred µm. Droplets evaporate as a function of their size, speed, chemical composition and how they are clustered together in the expiratory "jet". These dependencies arise because evaporation is driven by a difference in partial pressure and temperature at the surface of the evaporating substance and its environment. External influences on the evaporation include the room temperature, humidity and ventilation patterns. Large droplets fall to the ground or another surface before they completely evaporate. These form the droplet route of infection. Smaller droplets evaporate to become so small that they linger for several minutes in the air. These are called droplet nuclei and represent the airborne route of infection. Generally, the larger the droplet is, the quicker it will fall. As droplets become smaller, at some point the size will be such that the droplets evaporate before they hit the ground. Humidity can affect how long a droplet can remain suspended in the air, with higher humidity environments slowing evaporation and lower humidity environments accelerating evaporation and therefore, airborne transmission risk.

Embodiments of the present disclosure provide a tube and/or patient interface which may reduce the likelihood of aerosols containing pathogens and/or contaminants in the expiratory gas becoming dispersed from the patient airway by physically filtering a flow of expiratory gases flow through a filter before passage of the flow of expiratory gases from the patient interface into ambient air. This feature is aimed at reducing the likelihood of aerosolization of particles and/or reducing the likelihood of spread by slowing the outflow velocity of unfiltered pathogens in the expiratory gases.

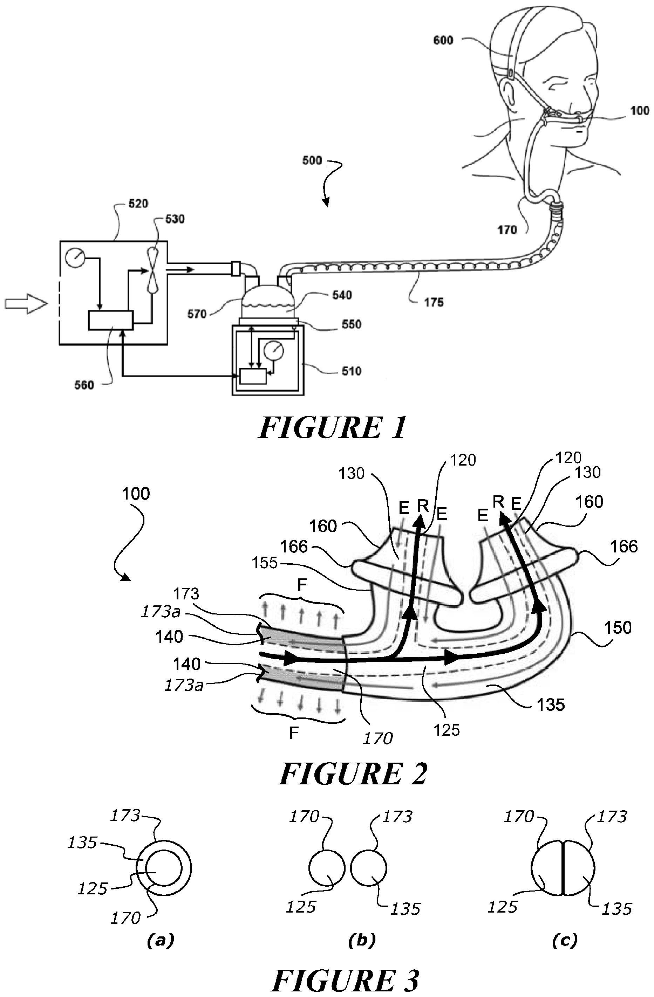

Referring initially to Figure 1, a respiratory support or assistance system 500 that may be used with the patient interfaces and tubes of the present disclosure is shown. In such a system, a patient 600 is supplied with a flow of gases through a patient interface 100. The flow of gases may be humidified. The patient interface 100 is retained in an operational position upon the patient's face using associated securement device, headgear or the like. The securement device provides a retention mechanism configured to retain the patient interface 100 on the patient's face in use.

The patient interface 100 is connected to a humidified gases transportation pathway or inspiratory conduit 170. The inspiratory conduit 170 defines an inspiratory lumen for directing a flow of gas toward the patient interface 100. The inspiratory conduit 170 is connected at one end to the patient interface 100 and at an opposing end to an outlet of a humidifier 510. The inspiratory conduit 170 may be connected to the humidifier 510 via an extension tube 175. The extension tube 175 and/or the inspiratory conduit 170 form a patient conduit adapted to connect to a gases source for delivering a gases flow to the patient 600.

The patient interface 100 is connected to an expiratory conduit 173 (Figure 2) that defines an expiratory lumen for directing a flow of expiratory gases from the patient. The expiratory conduit 173 is connected at one end to the patient interface 100.

The inspiratory conduit 170 and expiratory conduit 173 are together part of a tube 174 for delivery of gases to the patient interface 100 and for treatment of expiratory gases from the patient interface. In alternative configurations, the inspiratory conduit 170 may be provided by a first tube and the expiratory conduit 173 may be provided by a separate second tube.

The inspiratory conduit 170 is configured to direct a flow of gas toward at least one inspiratory lumen 120 of the patient interface, and the expiratory conduit 173 is configured to direct a flow of gas from at least one expiratory lumen 130 of the patient interface.

The extension tube 175, tube 174, and/or conduits 170, 173 can be part of the patient interface 100. Therefore, as used herein, "patient interface" can be not only the portion of the interface that contacts and/or interfaces with the patient, but also the extension tube 175, tube 174 that comprises the inspiratory conduit 170 that delivers the flow of gases to the patient interface and the expiratory conduit 173 that receives expiratory gases from the patient interface.

The humidifier 510 receives and humidifies gas supplied from a flow generator or respiratory gas source 520, which may include a blower 530. Alternatively or additionally, other gas sources may be used, such as a wall source with a flow meter. The humidifier 510 and gases source 520 may be integrated or may be in separate modules. The humidifier 510 may comprise a humidification chamber 570, filled with water or other liquid 540 and a heating means 550 for heating the water 540 to humidify the gas path through the humidifier 510. A controller 560 may be provided to control and possibly vary one or more properties of the supplied gas, including but not limited to the pressure profile of the gas, flow rate profiles of the gas at the patient interface 100, temperature and/or humidity of the gas. It will be appreciated that the control capabilities are dependent on the purpose and application of the respiratory support system 500.

The respiratory system 500 may be a high flow therapy apparatus or system. High flow therapy as discussed herein is intended to be given its typical ordinary meaning as understood by a person of skill in the art, which generally refers to a respiratory support system delivering a targeted flow of humidified respiratory gases via an intentionally unsealed patient interface with flow rates generally intended to meet or exceed inspiratory flow of a patient. Typical patient interfaces include, but are not limited to, a nasal or oral patient interface. Typical flow rates for adults often range from, but are not limited to, about fifteen litres per minute (LPM) to about seventy LPM or greater. Typical flow rates for paediatric patients (such as neonates, infants and children) often range from, but are not limited to, about one litre per minute per kilogram of patient weight to about three litres per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), among other common names. The flow rates used to achieve 'high flow' may be any of the flow rates listed below.

For example, in some configurations, for an adult patient "high flow therapy" may refer to the delivery of gases to a patient at a flow rate of greater than or equal to about 10 LPM, such as between about 10 LPM and about 120 LPM, or between about 10 LPM and about 100 LPM, or between about 15 LPM and about 95 LPM, or between about 20 LPM and about 90 LPM, or between 25 LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above. The flow therapy apparatus 10 can deliver any concentration of oxygen (e.g., fraction of delivered oxygen, FdO2), up to 100%, at any flowrate between about 1 LPM and about 100 LPM. In some configurations, any of the flowrates can be in combination with oxygen concentrations (FdO2) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the flow therapy apparatus 10 may include safety thresholds when operating in manual mode that prevent a user from delivering too much oxygen to the patient.

High flow therapy may be administered to the nares of a user and/or orally. High flow therapy may deliver gases to a user at a flow rate at or exceeding the intended user's peak inspiratory flow requirements. The high flow therapy may generate a flushing effect in the nasopharynx such that the anatomical dead space of the upper airways is flushed by the high incoming gases flow. This can create a reservoir of fresh gas available for each breath, while minimizing re-breathing of nitrogen and carbon dioxide. Meeting inspiratory demand and flushing the airways is additionally important when trying to control the patient's fraction of inhaled oxygen, FiO2. High flow therapy can be delivered with a non-sealing patient interface such as, for example, a nasal cannula. The nasal cannula may be configured to deliver breathing gases to the nares of a user at a flow rate exceeding the intended user's peak inspiratory flow requirements.

The term "non-sealing patient interface" as used herein can refer to an interface providing a pneumatic link between an airway of a patient and a gases flow source that does not completely occlude the airway of the patient. Non-sealed pneumatic links can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or both nares of the patient. For a nasal cannula the airway is through one or both of the nares.

Referring to Figure 2, a patient interface 100 for delivery of gas to a patient is shown. The patient interface 100 has at least one inspiratory element 155 comprising an inspiratory lumen 120 defining an inspiratory gas flow path for directing a flow of gas to a patient airway, via at least one of the nares. At least one expiratory element 155 comprising an expiratory lumen 130 defining an expiratory gas flow path directs a flow of expiratory gases from the patient. Provided in, or in fluid communication with, the expiratory gas flow path is a gas permeable body comprising a filter 140.

The filter 140 may be provided in any suitable location or component of patient interface provided that it is configured such that expiratory gases in the expiratory flow path are directed through the filter before exiting the patient interface and passage of the flow of expiratory gases from the patient interface and tube 140 into an ambient environment comprising ambient air, several examples of which are described in further detail below. The configuration may be such that the filter 140 at least partially treats the expiratory gases before exiting the patient interface 100 and tube 174. In some embodiments, treating the expiratory gases may involve mitigating release and/or reducing velocity and/or concentration of aerosols or other particle contaminants into the ambient air.

The patient interface 100 may include a device for mounting or otherwise attaching the apparatus on the patient. In some embodiments, a securement device (not shown) is provided to secure the patient interface to the patient and may include an elasticised or resilient strap, ties or an adhesive dermal pad for temporarily attaching at least part of the patient interface to the patient's skin thereby retaining the patient interface in an operable position. Alternatively, the securement device may comprise a cap/skull cap type headgear. Such headgear may comprise a plurality of straps or a continuous or meshed fabric or non-woven sheet forming a cap. Alternatively or additionally such headgear may comprise a combination of rigid or substantially rigid members and straps. The patient interface may comprise one or more connection features for coupling any of the securement devices listed. The one or more connection features may allow for permanent coupling of the securement device, or it may be removable or detachable.

As outlined above, the inspiratory conduit 170 is configured to direct a flow of gas toward at least one inspiratory lumen 120 of the patient interface, and the expiratory conduit 173 is configured to direct a flow of gas from at least one expiratory lumen 130 of the patient interface.

In the configuration shown in Figure 2, the patient interface comprises at least one inspiratory element 155 for directing a flow of gas to a patient airway, the or each inspiratory element comprising the at least one inspiratory lumen 120 through which said flow of gas is directed, and at least one expiratory element 155, the or each expiratory element comprising the at least one expiratory lumen 130 that provides an expiratory gas flow path.

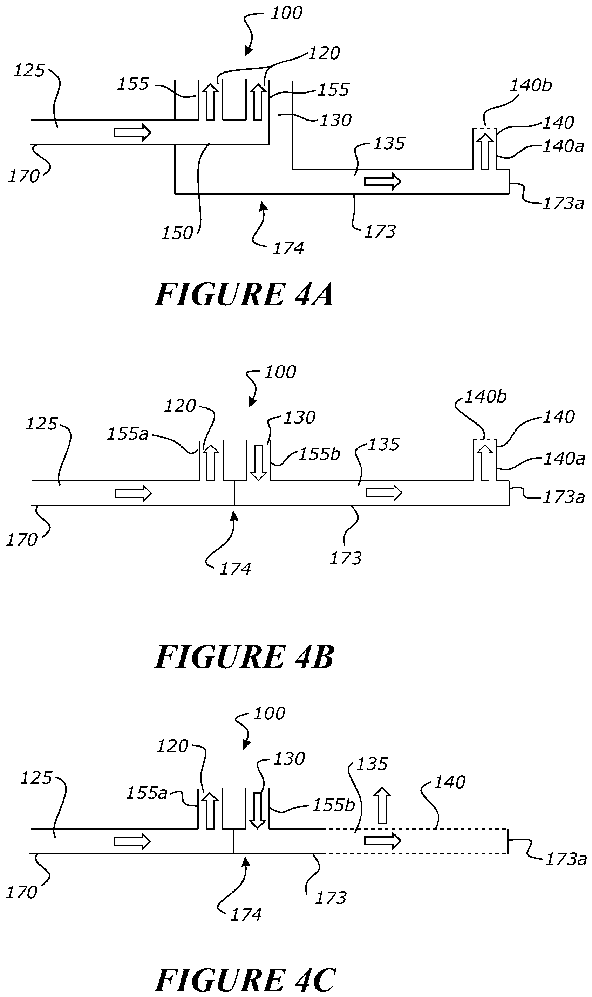

Depending on the configuration of the patient interface 100, the at least one inspiratory element comprises one or both of the first and second nasal elements 155, and the at least one expiratory element comprises one or both of the first and second nasal elements 155. These alternatives are described below with reference to Figures 4A to 4H.

In the embodiment of Figure 2, the patient interface 100 comprises first and second nasal elements 155 both configured to direct gas into and from a nare of the patient. Therefore, each nasal element 155 has an inspiratory lumen 120 and an expiratory lumen 130.

In the configuration shown, the first nasal element comprises a first nasal prong and the second nasal element comprises a second nasal prong.

In the embodiment of Figure 2, the expiratory lumen 130 defining the gas flow path of the expiratory element is provided through a head portion or cover 160 over the part of the nasal element 155 that defines the inspiratory lumen 120. In some embodiments, this part of the nasal element 155 may be described as a nasal prong, and the head portion or cover 160 is in the form of a cap or pillow. While the embodiment shown provides head portion or cover 160 as a separate element, it is to be understood that the head portion or cover could be integral with or part of the nasal element.

The head portion or cover 160 is sized and/or shaped to occupy the nare such that expiratory gases E from the nare are predominantly directed through the expiratory lumen 130 of the nasal element and through the expiratory conduit 173 to the filter 140. The external surface of the head portion or cover 160 abuts the internal wall of the nare such that expiratory gases are directed through it. If the nare is not fully occupied by the head portion or cover 160, some expiratory gas may flow around the nasal element. However, a significant proportion of expiratory gas will be directed through the expiratory conduit 173 and filtered by the filter 140.

In some embodiments, the head portion or cover 160 may be removable from the nasal prongs and replaceable.

In some embodiments, the head portion or cover 160 includes a skirt 166 configured to rest over the opening to the nare to improve occlusion and ensure substantially all expiratory gases from the nare are directed through the expiratory lumen 130.

In some configurations the nasal prongs are configured to substantially seal within the nares of the patient, and there is a permissible amount of leak from the mouth of the patient. In some configurations, the nasal prongs are unsealed with the nares of the patient, and there is a permissible amount of leak from the nares and mouth of the patient.

Figure 2 also schematically shows a cannula manifold 150 which provides a gas inflow path through manifold inspiratory lumen 125 to the inspiratory lumen 120 from an inspiratory conduit 170 of an inspiratory tube which is in fluid communication with a respiratory gas source. It is to be understood, however, that cannula manifold 150 may be omitted and the flow of respiratory gas R may be provided by conduit 170 directly connected or coupled with the inspiratory lumen 120 of the nasal prongs 155. Alternatively, the head portion or cover 160 may provide inspiratory lumen 120 in lieu of nasal prongs, for delivery of respiratory gas from cannula manifold 150. In such arrangement, the head portion or cover 160 has an internal membrane which defines inspiratory lumen 120 and is configured for coupling with cannula manifold 150 such that the inspiratory lumen in the cover is in fluid communication with manifold inspiratory lumen 125.

The cannula manifold 150 may be co-operably received in a cannula body that comprises the nasal prongs 155. Examples of cannula body configurations are described below.

In the configuration shown in Figure 2, each nasal prong provides both an inspiratory lumen and an expiratory lumen and the expiratory lumen may be parallel or coaxial with the inspiratory lumen. Each nasal prong is in fluid communication with the manifold inspiratory lumen 125 and manifold expiratory lumen 135 in cannula manifold 150.

Head portions or covers 160 are configured for coupling with the nasal prongs 155 and the cannula manifold 150 is configured for coupling with the cannula body such that the expiratory lumen 130 in the cover is in fluid communication with manifold expiratory lumen 135 which together form the expiratory gas flow path directing expiratory gases through the filter 140 before exiting the patient interface 100 and tube 174. In some embodiments of patient interface 100, the filter 140 may be located distally from the nasal elements.

The head portion or cover 160 may occupy the nare to limit or preclude release of expiratory gases which are not first passed through the filter 140. The head portion or cover 160 may be formed in part or in its entirety from any suitable material such as silicon, foam or other material. The head portion or cover 160 may be configured to be applied over the nasal prong or may be integrally formed the nasal prong so as to occlude or occupy the nare. In some embodiments, the head portion or cover 160 includes a skirt 166 configured to rest over the opening to the nare to improve occlusion and ensure all expiratory gases from the nare are directed through the filter 140. The filter 140 is provided further along the expiratory flow path and more distal the patient than the head portion or cover 160 is. The filter is therefore downstream in the expiratory flow path.

It is to be understood that while embodiments of the disclosure are described in the context of nasal cannula devices directing a flow of gas into both nares of the patient, the disclosure also contemplates a patient interface which directs gas flow into a single nare and allows expiratory gases flow to exit from the other nare.

In the example shown in Figure 2, the filter 140 is configured as a sheath formed around at least part or all of an expiratory conduit 173 that is in fluid communication with the manifold expiratory lumen 135. In alternative configurations, the filter 140 may form the expiratory conduit 173 or part thereof, or the filter 140 may be a separate component that is in fluid communication with the expiratory conduit 173. The expiratory conduit 173 may be integrally formed with the patient interface, e.g. the cannula manifold 150, or may be coupled to the patient interface.

The filter 140 may comprise a filter tube and filter member. The filter tube, and thereby the filter 140, may be provided as a separate component that can be utilised in a respiratory circuit if needed. For example, the filter tube may be connectable to an expiratory port of the cannula manifold 150, or may be connectable to an expiratory conduit 173 that receives expiratory gases from the cannula manifold 150. The filter tube can be provided as a separate consumable that can be connected to any respiratory circuit that has the capability to channel at least a portion of expiratory gases.

In some configurations, the filter tube provides the inspiratory lumen 170 and the expiratory lumen 173a. The filter tube may have a first open end and a second open end. The first open end may be connectable to a gas source or humidified gas source, and the second open end may be connectable to a patient interface 100, e.g. to the cannula manifold 150. The connection(s) could be via any suitable configuration, for example a standard medical taper or a proprietary connector of any suitable configuration.

Although not shown in Figure 2, a portion of the filter tube upstream of the filter 140 may comprise a wall that is substantially non-permeable. That portion will be adjacent a patient's face in use, and closer to the patient's face than the filter 140. That minimises discomfort from expiratory gases flow through the filter contacting patient skin.

The inspiratory conduit 170 is in fluid communication with the manifold inspiratory lumen 125. The inspiratory conduit 170 may be integrally formed with the patient interface, e.g. cannula manifold 150, or may be coupled to the cannula manifold.

As shown Figure 2, the inspiratory conduit 170 and the expiratory conduit 173 may be on a single limb, such that the that the inspiratory conduit 170 and the expiratory conduit 173 both extend in one direction from the patient interface 100 in use. For example, the inspiratory conduit 170 and the expiratory conduit 173 may both extend from the right side of the patient interface in use or alternatively may both extend from the left side of the patient interface in use. As described in more detail below, the cannula manifold 150 may be reconfigurable relative to the cannula body to enable the inspiratory conduit 170 and the expiratory conduit 173 to extend from either side of the patient interface in use.

In the configuration shown in Figure 2, the inspiratory conduit 170 and the expiratory conduit 173 are coaxial. In an alternative configuration, the inspiratory conduit 170 and the expiratory conduit 173 may be substantially parallel or alternatively may both extend from one side of the patient interface in use, but at a non-parallel angle.

Figure 3 shows exemplary configurations of inspiratory conduit 170 and expiratory conduit 173 which may be used, including a co-axial configuration where the inspiratory conduit 170 is inside the expiratory conduit 173 (Figure 3(a)), a separate side-by-side inspiratory conduit 170 and expiratory conduit 173 which may be parallel for example and which are provided by a separate inspiratory tube and a separate expiratory tube (Figure 3(b)), and a conjoined inspiratory conduit 170 and expiratory conduit 173 (Figure 3(c)).

When a separate expiratory tube is provided, the expiratory tube can be connected to any system that can channel or divert at least a portion of the expiratory gases flow into the expiratory tube to filter the flow of expiratory gases before passage of the flow of expiratory gases into ambient air.

In the coaxial configuration, the inspiratory conduit 170 will typically be an inner conduit and the expiratory conduit 173 will typically be an outer conduit. The inner conduit may be sealed from the outer conduit or alternatively the inner conduit may be breathable. Hereinafter, throughout the description, a material that allows the passage of water molecules through a monolithic wall of the material via the solution-diffusion mechanism, without allowing the bulk passage of liquid water or bulk flow of respiratory gases all the way through the wall is described as a "breathable" material. It should be appreciated by one of skill in the art that the water molecules in the wall are molecularly dispersed in the media, and are therefore without a state (solid, liquid, or gas), although they are sometimes referred to in the art as vapour (e.g. the rate of transfer is often referred to as a water vapour transmission rate or the like). It should further be appreciated that a monolithic wall does not contain open channels or through holes from one major surface to another, such that viruses could be carried through such channels or holes alongside air or liquid water drops via the pore flow mechanism. The outer lumen may act to insulate the inner lumen to reduce the incidence of condensation in the inner inspiratory conduit.

Manifold expiratory lumen 135 is in fluid communication with pores of the gas permeable body that provides the filter 140 such that expiratory gases E are directed from the nares through manifold body 150 and then through gas permeable body 140 such that treated or filtered expiratory gases F are released from the patient interface 100 directly to ambient air. Alternatively or additionally, the filter 140 could be a component within the expiratory conduit 173 or integral with the tube from which it is formed. For instance, the wall of the expiratory conduit 173 may comprise the filter. In one example, the wall of the expiratory conduit 173 in the filter region 140 may be formed from, e.g. extruded from, gas permeable filter material, with the internal respiratory gas delivery channel or lumen defined by an internal gas impermeable membrane which maintains separation of inspiratory and expiratory gases.

The filter 140 is configured to capture aerosols entrained within the flow of expiratory gases, and may further be configured to capture and substantially eliminate pathogens.

In some embodiments, it is envisaged that the filter 140 provided by gas permeable body comprising a filter material which is porous i.e. permeable to expiratory gases. Thus, "pores" as used throughout the description of the illustrated embodiments refer to the spaces or gaps in the filter material through which expiratory gases travel. In some instances, a porosity or filter material coarseness which allows for normal breathing is sufficient. However, in circumstances where the patient is receiving NHF through patient interface 100, the filter material ideally provides for higher expiratory gas flow rates to avoid compromising treatment or causing harm to the patient. Thus, the filter material is ideally sufficiently coarse to limit the increase in resistance to flow due to expiratory gases being directed through the gas permeable body while also being effective in filtering or intercepting to reduce the concentration of particles in the flow of expiratory gases, or at the very least reducing the velocity of particles in the expiratory gases.

It may be desirable to utilise filter material formed of or containing a gas permeable body comprising an N95 or higher (e.g. N99) rated or HEPA filter material to reduce transmission risk. The filter material may include one or more materials selected from a group comprising but not limited to fibrous, porous knitted, mesh, woven foam, textile, non-textile, foam or another filter material.

A length and/or surface area of the filter 140 in the wall of the expiratory conduit 173 may be configured to reduce a resistance to flow of gases through the filter 140. For example, the length of the filter 140 in the wall of the expiratory conduit 173 may be between about 160 mm and about 295 mm. The surface area of the filter may, for example, be up to about 13,270 mm². In some configurations, the surface area of the filter 140 may, for example, be between about 6,535 mm² and about 12,050 mm².

In some configurations, the length of the expiratory conduit may be between about 325 mm and 390 mm. In some configurations, the inner diameter of the expiratory conduit 173 may be about 13 mm. Any other suitable configurations may be used.

In some embodiments, a filter material having a larger surface area of filter media may reduce the resistance to flow attributable to expiratory gases being directed through the gas permeable body.

A portion of the expiratory conduit 173 upstream of the filter 140 (i.e. closer to the patient interface than the filter) may have a wall that is substantially non-permeable.

The portion of the expiratory conduit 173 upstream of the filter may have a length of at least about 80 mm to avoid contact of the filter with the patient's face to minimise discomfort from expiratory gases flow through the filter contacting patient skin. The portion of the expiratory conduit 173 may have a length of up to about 195 mm.

An end 173a of the expiratory conduit 173 that is downstream of the filter 140 will typically be closed and gas-impermeable to inhibit the flow therethrough of expiratory gases and to direct the expiratory gases through the filter 140.

In various embodiments, at least part of the patient interface may be thermally conductive. For example, an inspiratory lumen directing gas into the patient's airway e.g. through nasal elements 155, may contain a thermally conductive material and is configured to facilitate warming of gas delivered to the patient. The gas permeable body that provides the filter 140 may be thermally conductive which may reduce resistance to flow though the gas permeable body, particularly when they are dampened or become dampened with use.

The tubes 174 and conduits 170, 173 described herein provide gases to the patient interface 100 and/or are provided in fluid communication or in connection with a cannula manifold 150, 1150, 2150, 3150, 4150 or cannula manifold assembly.

Such a tube and/or conduit may be formed of a breathable and flexible type.

Such a tube or conduit, and in particular at least the expiratory conduit 173, may comprise a structure to prevent or minimise collapse or constriction of the expiratory lumen 135 in the expiratory conduit. The structure may comprise one more of: corrugations, pleats, helical wrap, ribs, or supporting features.

In one configuration, the tube may be formed by winding a filter material membrane helically onto a former. A helical supporting rib, provided in a semi-molten state, is laid on the overlap between adjacent turns. The heat from the helical supporting rib bonds the two adjacent strips with the rib forming a flexible resilient conduit once cooled. The helical supporting rib could be chosen from a range of thermoplastics which provide sufficient structural and processing properties. The melt temperature of the helical supporting rib and the filter material membrane should be similar to aid processing when formed in this manner. An example polymer is Arnitel^{®} EM550 which is a thermoplastic co-polyester. The forming may otherwise be as described in US 6,769,431. The contents of that specification are incorporated herein in their entirety by way of reference.

The tubes 174 and/or conduits may be crush-resistant and/or may be made of a material that reduces or may minimise noise generation when the conduit is moved or bent, such noises are sometimes being referred to as a "crinkling" sound. For example, the tube may comprise of an elongate film spirally wrapped with an elongate reinforcing member to form the tube's lumen. The tube may also be of the type which is extruded to form the conduit. In various embodiments, breathable material(s) can be used in the construction of the inspiratory conduit to assist with expelling of any accumulated condensate (e.g. from "rain-out") in the conduit.

In various embodiments, the reinforcing member may help to prevent or reduce crushing and/or kinking of the tube, or at least the potential for crushing and/or kinking during use. The lumen of the conduit may comprise of an inner bore that is substantially smooth so as to reduce resistance to flow and/or minimise surface features upon which condensation may accumulate or pool.

It is preferable that the tubing used for the inspiratory conduit 170 and the expiratory conduit 173 is: resistant to crushing, resistant to restrictions in flow when bent (e.g., increased resistance to flow <50% when bent around a 1 inch cylinder), resistant to kinking, resistant to changes in length/volume under fluctuating internal pressure (e.g., resistance to compliance), resistant to leaking (e.g., <25ml/min @6kPa), have low flow resistance (e.g., increase in pressure @ max. rated flow <0.2kPa), electrically safe (e.g., resistant to sparks in the tubing) given an operating environment that may be oxygen-rich.

The patient interface 100 and tube 174 for delivering gases to the patient interface 100 and for treatment of expiratory gases can have many different configurations, some of which are shown schematically in Figures 4A to 4H for example. Unless described differently below, the features, functionality, and options are the same as for the configuration of Figure 2, and like reference numerals indicate like parts. Any of the patient interfaces and tubes described herein can be used in a respiratory support or assistance system, such as that described with reference to Figure 1 for example.

As mentioned above in relation to Figure 3, the inspiratory conduit 170 and expiratory conduit 173 of the configurations of Figures 4A-4H could have any suitable configuration, such as a co-axial configuration where the inspiratory conduit 170 is inside the expiratory conduit 173 (Figure 3(a)), a separate side-by-side inspiratory conduit 170 and expiratory conduit 173 which may be parallel (Figure 3(b)), and a conjoined inspiratory conduit 170 and expiratory conduit 173 (Figure 3(c)).

In each configuration, the tube 174 comprises an inspiratory conduit 170 defining for directing a flow of gas toward the patient interface 100 and an expiratory conduit 173 for directing a flow of gas from the patient interface 100. The filter 140 is in fluid communication with, or part of, the expiratory conduit 173, the filter 140 configured to filter the flow of expiratory gases from the patient interface 100 before passage of the flow of expiratory gases from the patient interface 100 into ambient air. In some configurations, an exterior surface of the filter 140 is in direct communication with ambient air, to deliver filtered expiratory gases to ambient air.

In some configurations, expiratory gases flow may be directed, filtered, and passed to the ambient air passively; that is, no active suction is applied to the expiratory conduit 173. In other configurations, active suction may be provided to pull gas flow through the expiratory conduit 173 and/or filter 140. In some configurations, simultaneous positive and negative flows may be used. For example, in addition to the use of a positive gas flow source to deliver gases to an airway of the patient, a negative gas flow source may be provided to create a gas flow that substantially progresses in a direction away from the airway of the patient via the expiratory conduit 173 and/or filter 140. The negative gas flow source may comprise a flow generator such as a blower. The features and functionality of the simultaneous positive and negative flows may be as described in PCT/NZ2015/050044 (published as WO 2015/156690) and US patent no. 10,610,653. The contents of those specifications are incorporated herein in their entirety by way of reference.

The tubes 174 may be provided in combination with the patient interfaces 100 or alternatively may be provided separately for retrofitting, repair, or replacement purposes. The components described herein may be provided in kit form to enable assembly together of one or more of the patient interface, tube, and/or filter.

In some configurations, the inspiratory conduit 170 may be provided as an inspiratory tube that is separate from an expiratory tube that forms the expiratory conduit 173.

The tubes 174, filters 174, and patient interfaces 100 can be used in a respiratory support or assistance system 500 such as that of Figure 1 for example.

In the configuration of Figure 4A, rather than being on a single limb, the inspiratory conduit 170 extends from one side of the patient interface 100 in use and the expiratory conduit 173 extends from the opposite side of the patient interface in use.

In this configuration, as in the configuration of Figure 2, each nasal element 155 provides both a nasal element inspiratory lumen 120 and a nasal element expiratory lumen 130. Alternatively, the patient interface 100 could be configured to provide a unidirectional flow wherein only one nasal element provides the nasal element inspiratory lumen 120 and only the other nasal element provides the nasal element expiratory lumen 130. Both alternatives (unidirectional and bidirectional) are possible for any of the configurations described herein.

In the configuration of Figure 4A, the expiratory conduit 173 is coupled to a filter component that comprises the filter 140. The filter component comprises a filter housing 140a that is in fluid communication with a downstream portion of the expiratory conduit 173 and a filter member 140b. The filter member 140b may be any of the materials described above in relation to Figure 2.

Alternatively, the wall of the expiratory conduit 173 may comprise the filter 140 as described above.

A portion of the expiratory conduit 173 upstream of the filter 140 and proximal to the patient interface may comprise a wall that is substantially non-permeable to minimise discomfort from expiratory gases flow through the filter contacting patient skin.

In the configuration of Figure 4B, the patient interface 100 is configured to provide a unidirectional flow wherein only one nasal element 155a provides the nasal element inspiratory lumen 120 and only the other nasal element 155b provides the nasal element expiratory lumen 130.

In this configuration, the expiratory conduit 173 is coupled to a filter component that comprises the filter 140, as in the configuration of Figure 4A. The wall of a portion of the expiratory conduit upstream of the filter and proximal to the patient interface may be substantially non-permeable. That is the portion of the expiratory conduit that is likely to contact the patient in use.

In the configuration of Figure 4C, the patient interface 100 is configured to provide a unidirectional flow wherein only one nasal element 155a provides the nasal element inspiratory lumen 120 and only the other nasal element 155b provides the nasal element expiratory lumen 130.

In this configuration, the wall of the expiratory conduit 173 comprises the filter 140, as in the configuration of Figure 2. The wall of the expiratory conduit upstream of the filter may be substantially non-permeable.

In the configurations of Figures 4B and 4C, the patient interface 100 comprises a first nasal element 155a to be provided in fluid communication with a patient's first nare, and an associated first conduit 170 provided for an independent and/or dedicated fluid connection with the first nasal element 155a. The patient interface 100 comprises a second nasal element 155b to be provided in fluid communication with a patient's second nare, and an associated second conduit 173 provided for an independent and/or dedicated fluid connection with the second nasal element 155b. One of the first nasal element 155a with said associated first conduit 170, or the second nasal element 155b with said associated second conduit 173, is configured as a sole inspiratory line or conduit for delivery of a source of gases to one or a first of the patient's nares. The other of the first nasal element 155a with said associated first conduit 170, or the second nasal element 155b with said associated second conduit 173, is configured as a sole expiratory line or conduit for receiving gases from the other one or a second of the patient's nares. The patient interface 100 comprises a partition or other physical separation between the first nasal element 155a and the second nasal element 155b. The partition or other physical separation provides for a mechanical fluid disconnection between a fluid flow path of the first nasal element 155a and a fluid flow path of the second nasal element 155b.

In some configurations, the inspiratory nasal element 155a can become the expiratory nasal element and vice versa. In particular, the gases flow can be selectively directed to either the first nasal element 155a or the second nasal element 155b, the selected nasal element becoming the dedicated inspiratory element and the other nasal element becoming the dedicated expiratory element.

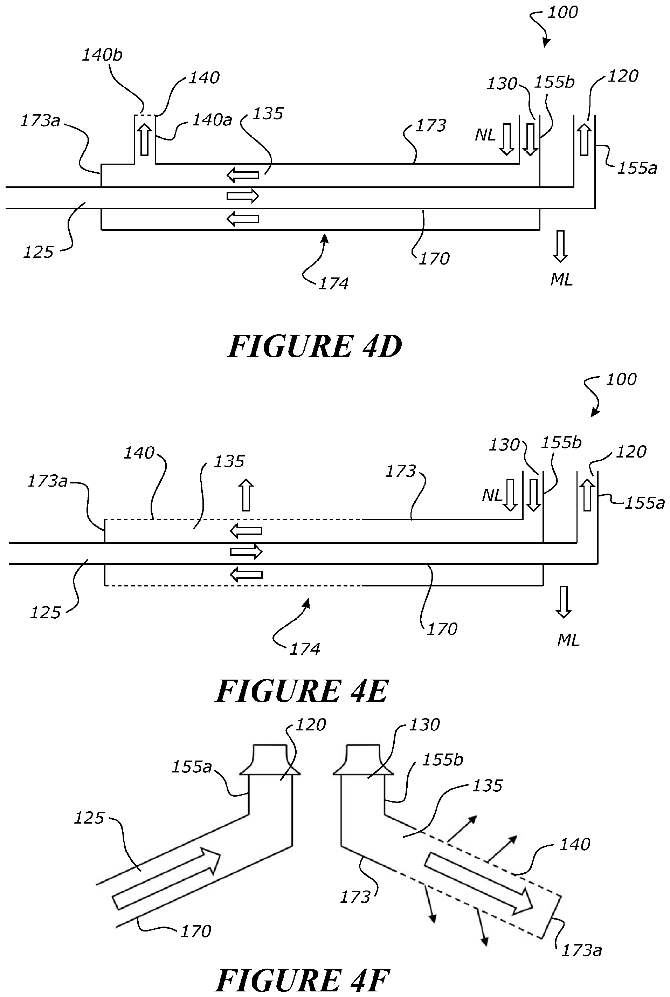

In the configuration of Figure 4D, the patient interface 100 is configured to provide a unidirectional flow wherein only one nasal element 155a provides the nasal element inspiratory lumen 120 and only the other nasal element 155b provides the nasal element expiratory lumen 130.

The inspiratory conduit 170 and the expiratory conduit 173 are on a single limb and are coaxial, as per the embodiment of Figure 2.

The expiratory conduit 173 is coupled to a filter component that comprises the filter 140, as per Figure 4A.

As indicated by arrows NL and ML in Figure 4D, in any of the configurations described herein, some amount of nasal leak NL and/or mouth leak ML may be permissible without adversely affecting the functioning of the technology. That is, the nasal elements 155a, 155b do not need to completely seal in the nares of the patient, and the patient may be able to expel a portion of their expiratory gases through their mouth. However, it is envisaged that the majority of the expiratory gases from a patient will be directed to pass through the filter 140 before exiting to ambient air.

For example, more than 50%, at least 55%, at least 60%, at least 65%, at least 70%, at least 75%, at least 80%, at least 85%, at least 90%, at least 95%, or substantially 100% of the expiratory gases from a patient may be directed to pass through the filter 140 before exiting to ambient air.

In the configuration of Figure 4E, the patient interface 100 is configured to provide a unidirectional flow wherein only one nasal element 155a provides the nasal element inspiratory lumen 120 and only the other nasal element 155b provides the nasal element expiratory lumen 130.

The inspiratory conduit 170 and the expiratory conduit 173 are on a single limb and are coaxial, as in the embodiment of Figure 2.

In this configuration, the wall of the expiratory conduit 173 comprises the filter 140, as in the configuration of Figure 2. The wall of the expiratory conduit upstream of the filter may be substantially non-permeable.

In the configuration of Figure 4F, the patient interface 100 is configured to provide a unidirectional flow wherein only one nasal element 155a provides the nasal element inspiratory lumen 120 and only the other nasal element 155b provides the nasal element expiratory lumen 130.

In this configuration, the wall of the expiratory conduit 173 comprises the filter 140, as in the configuration of Figure 4C. The wall of the expiratory conduit upstream of the filter may be substantially non-permeable. In an alternative configuration, the expiratory conduit 173 is coupled to a filter component that comprises a filter 140, as in the configuration of Figure 4B.

In configuration of Figure 4F, the nasal elements 155a, 155b comprise separate, unconnected nasal elements. The nasal elements may be sealing or non-sealing.

As outlined above for the configuration of Figure 4B and 4C, in the configuration of Figure 4F, the patient interface 100 comprises a first nasal element 155a to be provided in fluid communication with a patient's first nare, and an associated first conduit 170 provided for an independent and/or dedicated fluid connection with the first nasal element 155a. The patient interface 100 comprises a second nasal element 155b to be provided in fluid communication with a patient's second nare, and an associated second conduit 173 provided for an independent and/or dedicated fluid connection with the second nasal element 155b. One of the first nasal element 155a with said associated first conduit 170, or the second nasal element 155b with said associated second conduit 173, is configured as a sole inspiratory line or conduit for delivery of a source of gases to one or a first of the patient's nares. The other of the first nasal element 155a with said associated first conduit 170, or the second nasal element 155b with said associated second conduit 173, is configured as a sole expiratory line or conduit for receiving gases from the other one or a second of the patient's nares. The patient interface 100 comprises a partition or other physical separation between the first nasal element 155a and the second nasal element 155b. The partition or other physical separation provides for a mechanical fluid disconnection between a fluid flow path of the first nasal element 155a and a fluid flow path of the second nasal element 155b.

In the configuration of Figure 4F, physical separation between the fluid flow paths of the first nasal element 155a and the second nasal element 155b is by physical independence or disconnection of the nasal elements 155a, 155b and/or associated conduits 170, 173.

In some configurations, the inspiratory nasal element can become the expiratory nasal element and vice versa. In particular, the gases flow can be selectively directed to either the first nasal element 155a or the second nasal element 155b, the selected nasal element becoming the dedicated inspiratory element and the other becoming dedicated expiratory element.

With physically separate nasal delivery elements, should the filter 140 need replacing, the expiratory nasal element can be replaced without needing to replace the inspiratory nasal element.

The patient interface 100 can be held in place on a patient face's by suitable arrangement, such as headgear, straps and/or dermal patch(es) for example.

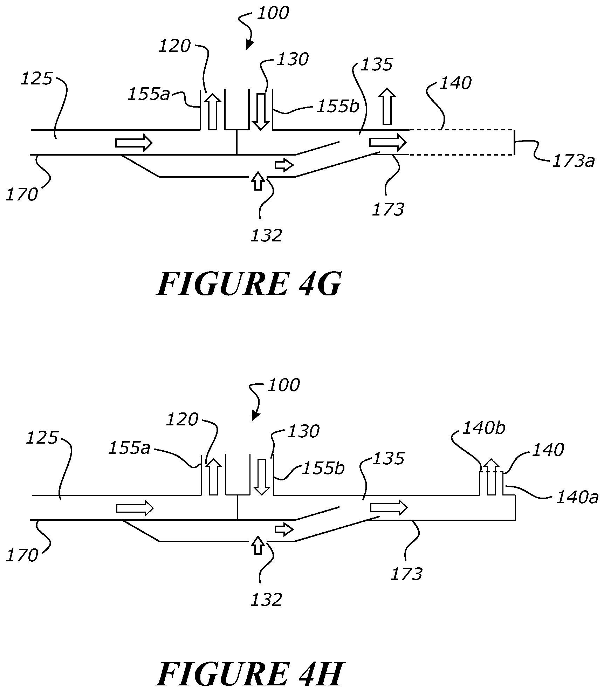

In the configuration of Figure 4G, the patient interface 100 is configured to provide a unidirectional flow wherein only one nasal element 155a provides the nasal element inspiratory lumen 120 and only the other nasal element 155b provides the nasal element expiratory lumen 130. The configuration is similar to that of Figure 4C.

The patient interface 100 comprises a gases port 132 for entrainment of expiratory gases from a patient's nose and/or a patient's mouth that are not directed into the expiratory nasal element. The gases port 132 may be configured to be positioned proximal to a patient's mouth in use of the patient interface.

The gases port 132 is open to atmosphere/ambient.

The gases port 132 is in fluid communication with, or opens into, the expiratory conduit 173 and/or expiratory lumen 135.

A flow of expiratory gases along the expiratory conduit 135 can create a negative pressure at the gases port 132 that acts to entrain gases from the patient's mouth and/or the patient's nose that have not been directed into the expiratory prong 155b.

The gases port 132 is positioned and oriented such that it can entrain at least some of expiratory gases from the patient's mouth and/or the patient's nose that are not directed into the expiratory lumen 135 via the expiratory nasal element 155b.

The gases port 132 is positioned and oriented such that a flow of expiratory gases from the patient's mouth and/or the patient's nose are moved in a direction from the gases port 132, into the expiratory lumen 135, and to the filter 140.

The effect of the entrainment can be increased by increasing a flow rate of gases that are delivered into the patient interface 100 through the inspiratory conduit 170, thereby increasing a flow rate of gases that pass along the expiratory conduit 173 to entrain more gases.

Active suction can be applied at a point along the expiratory conduit 173, for example the distal end of the expiratory conduit 173, to increase the entrained gases. Active suction can be applied by, for example, a secondary flow generator or vacuum, such as vacuum available from wall outlets in most hospitals.

In the configuration of Figure 4H, the expiratory conduit 173 is coupled to a filter component that comprises a filter 140, as in the configuration of Figure 4B. The features and functioning of the configuration of Figure 4H are otherwise as described for Figure 4G.

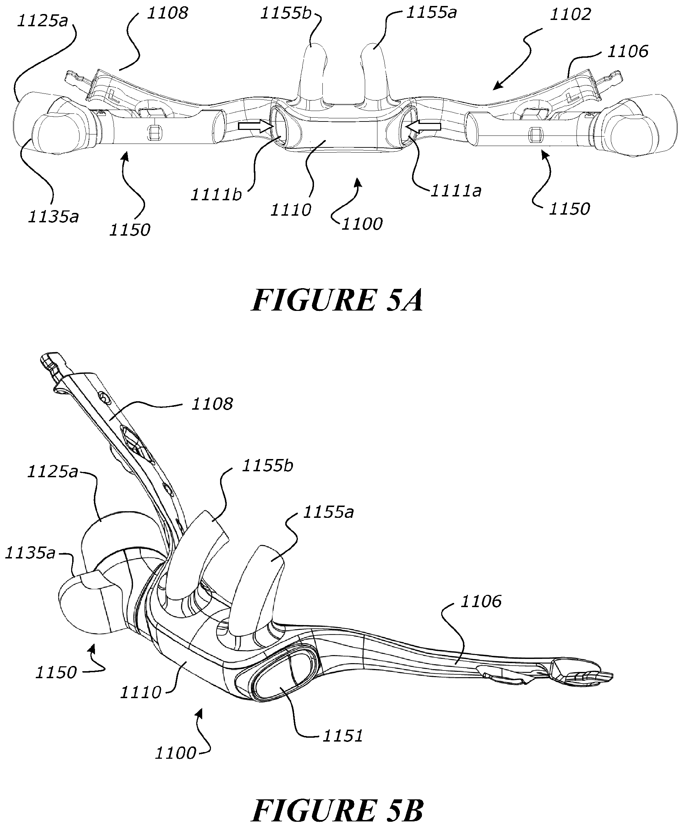

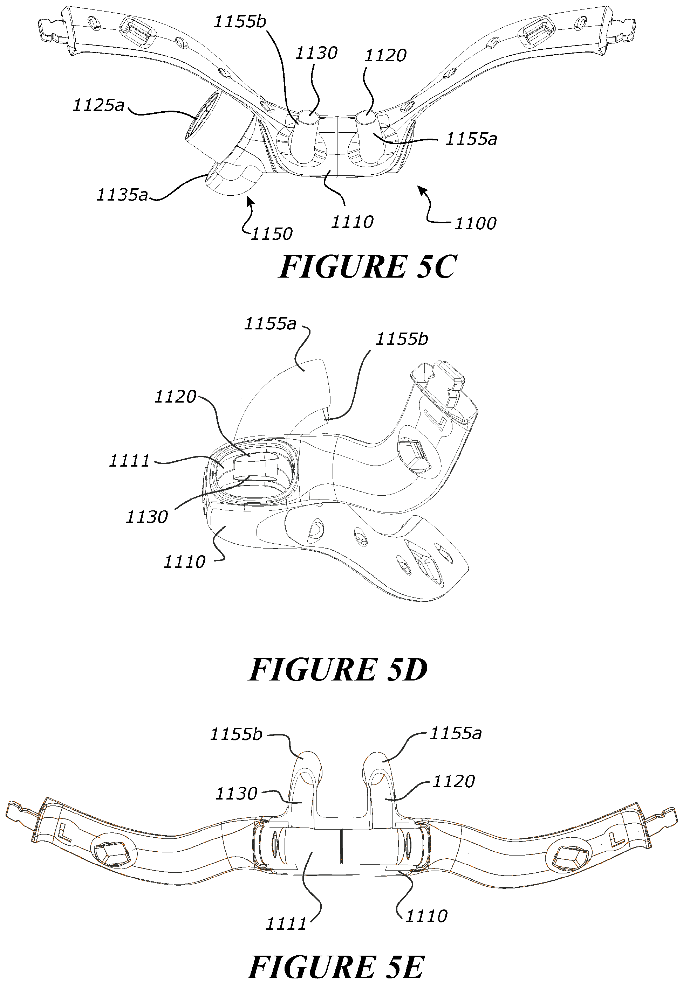

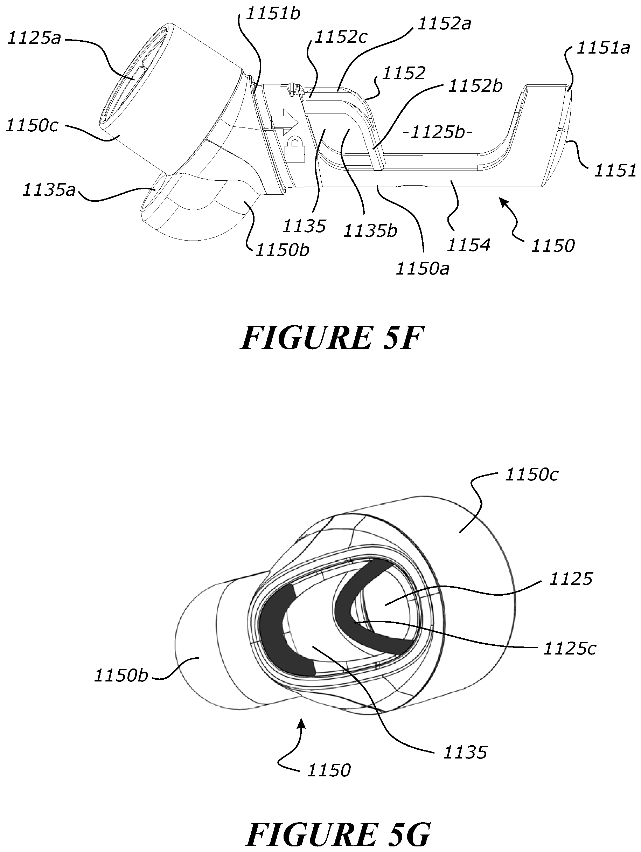

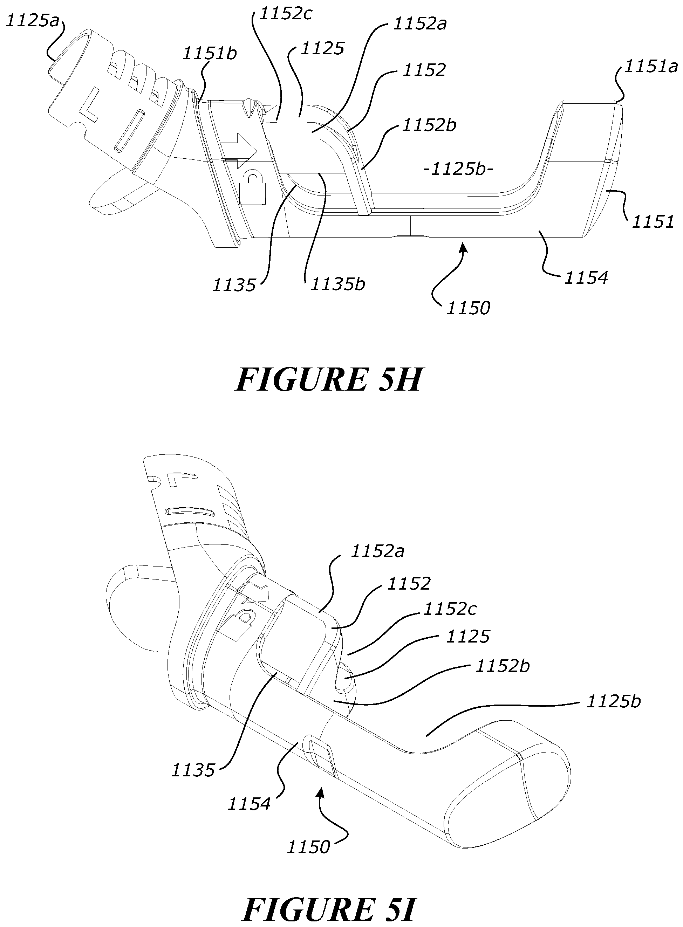

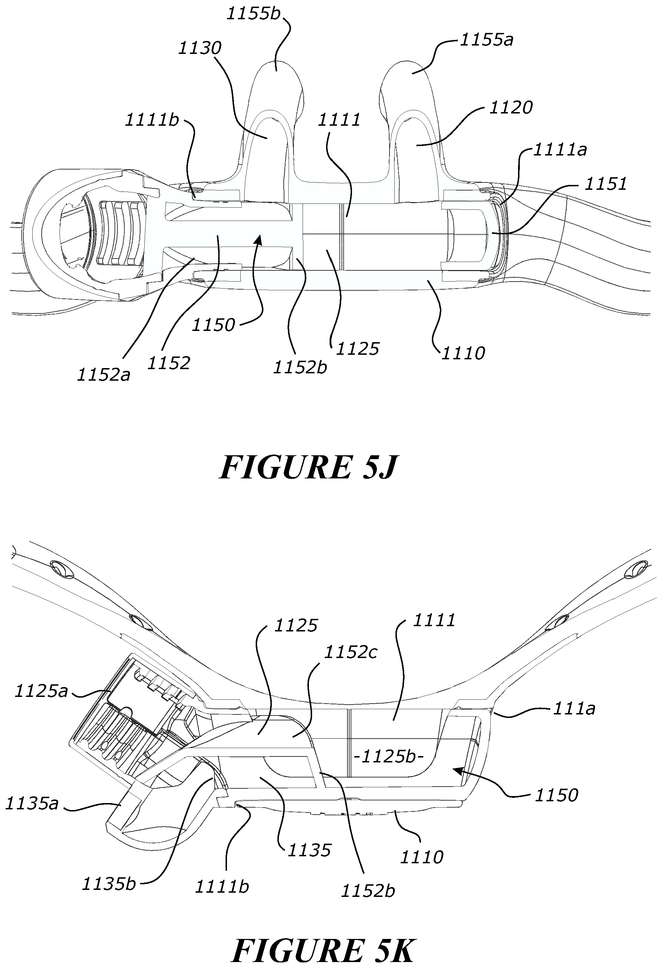

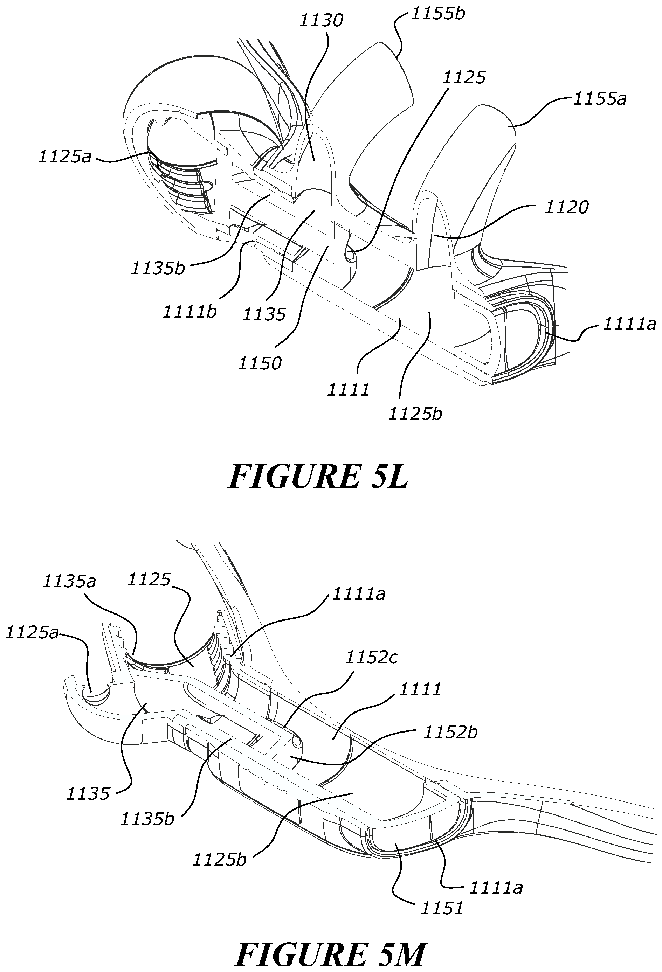

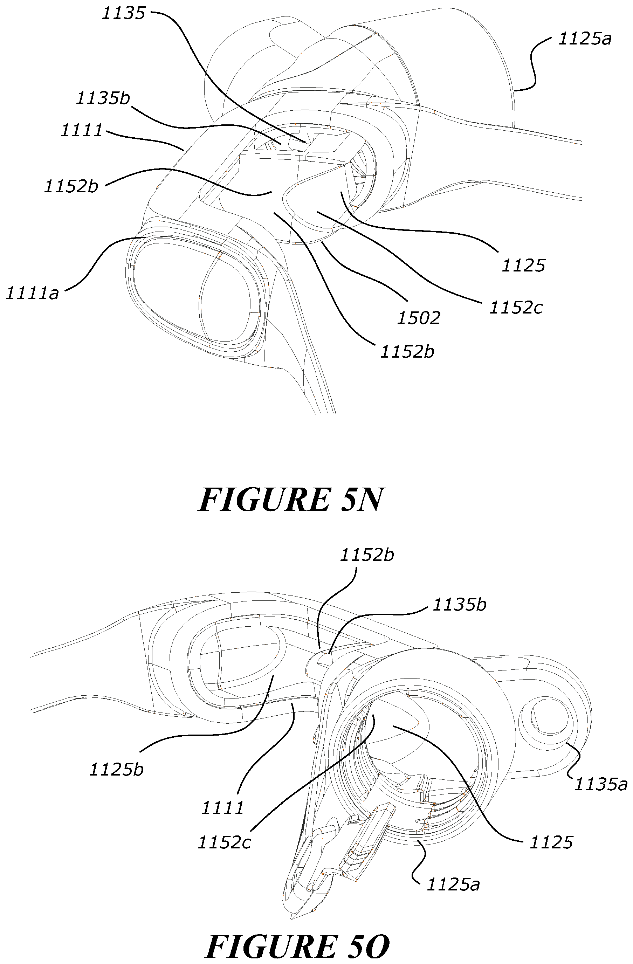

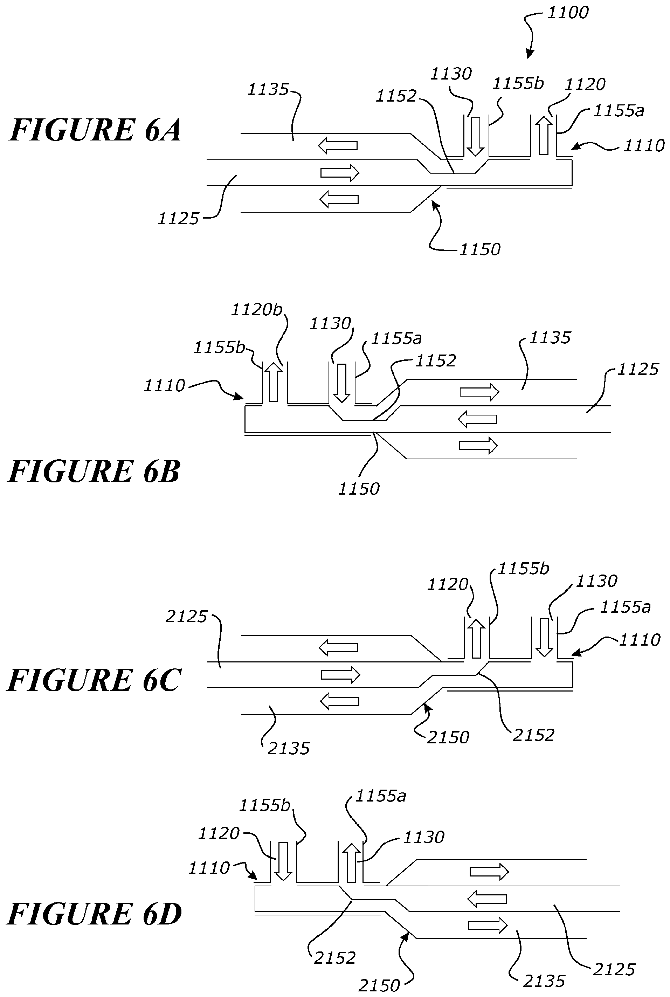

As mentioned above, the cannula manifold may be reconfigurable relative to the cannula body. Figures 5A to 5O show an alternative configuration patient interface 1100 that enable the cannula manifold 1150 to be reconfigured relative to the cannula body 1110 to select which nasal element provides the inspiratory element and which nasal element provides the expiratory element, and Figures 6A and 6B schematically show the two configurations.

The patient interface 1100 may have any one or more of the features, functionality, and options outlined for the embodiments described above, and like reference numerals indicate like parts with the addition of 1000.

A frame portion 1102 of the interface 1100 comprises a first side arm 1106 and a second side arm 1108. The side arms 1106, 1108 may comprise headgear retaining mechanisms adapted to hold headgear. The retaining mechanism may comprise buckles, connectors, or any other suitable features for engaging with the headgear.

The patient interface 1100 comprises a manifold inspiratory lumen 1125 that is in fluid communication with a manifold inlet 1125a and a manifold expiratory lumen 1135 that is in fluid communication with a manifold outlet 1135a.

The frame portion 1102 comprises a cannula body 1110.

The cannula body 1110 has a first nasal element 1155a and a second nasal element 1155b, which may comprise nasal prongs of the type described above for example. The nasal elements 1155 may be integrally formed with the cannula body 1110, or could alternatively be formed as part of an elastomeric component. The elastomeric component may be overmould component that is overmoulded onto the cannula body, with an underlying portion of the cannula body including cavity 1111 being more rigid. The body may comprise a polymer material such as polypropylene for example, and the elastomeric component may comprise thermoplastic elastomer for example.

With reference to Figures 5C to 5O, the cannula manifold 1150 comprises a flow directing member 1152. The cannula manifold 1150 is reconfigurable relative to the cannula body such that in a first configuration the flow directing member 1152 is arranged to direct gas flow from the manifold inspiratory lumen 1125 to the first nasal element 1155a and to direct gas flow from the second nasal element 1155b to the expiratory lumen 1135 (as shown in Figure 6A for example), and in a second configuration the flow directing member 1152 is arranged to direct gas flow from the manifold inspiratory lumen 1125 to the second nasal element 1155b and to direct gas flow from the first nasal element 1155a to the manifold expiratory lumen 1135 (as shown in Figure 6B for example).

The cannula manifold 1150 is configured to be inserted into the cavity 1111 of the cannula body 1110. In particular, the cannula manifold 1150 is configured to be inserted into the cavity 1111 of the cannula body 1110 in the first configuration and in the second configuration.

The cannula body 1110 has a cavity 1111 for receipt of the cannula manifold 1150. The cannula body 1110 has a first open end 1111a for insertion of the cannula manifold 1150 in a first configuration, and a second open end 1111b for insertion of the cannula manifold 1150 in a second configuration.

The cavity 1111 extends transversely across the cannula body 1110 from the first open end 1111a at one side of the cannula body 1110 to the second open end 1111b at the other side of the cannula body 1110.

The cavity 1111 acts as a gases chamber of the cannula body 1110. The cavity 1111 is in fluid communication with a nasal element inspiratory lumen 1120 and with a nasal element expiratory lumen 1130, as shown in Figure 5J and 5L for example.

The cannula manifold 1150 may be received in the cavity 1111 in the two configurations - either via the first open end 1111a or via the second open end 1111b. If a user desires the tube 174 to extend from the patient interface 1100 on the right-hand side then the cannula manifold 1150 may be inserted into the cavity 1111 via the first open end 1111a. Alternatively, if the user desires the tube 174 to extend from the patient interface 1111b on the left-hand side then the cannula manifold 1150 may be inserted into the cavity 1111 via the second open end 1111b.

This means that the same cannula manifold 1150 can be used irrespective of which side of the patient interface 1100 the tube 174 will extend from, thereby reducing the number of separate components that are required.

The first configuration corresponds to the cannula manifold 1150 being inserted into cavity 1111 of the cannula body from a first side of the cannula body 1110, and the second configuration corresponds to the cannula manifold 1150 being inserted into the cavity 1111 of the cannula body from a second side of the cannula body 1110.

Figures 5B, 5C, 5L to 5O, and 6A show the first configuration where the cannula manifold 1150 is inserted into the right side of the cannula body 1110 (relative to a patient) via the first open end 1111a so that the inspiratory lumen 1125 and expiratory lumen 1135 are accessible from a right side of the patient interface. Figure 6B schematically shows the second configuration where the cannula manifold 1150 is inserted into the left side of the cannula body 1110 (relative to a patient) via the second open end 1111b so that the inspiratory lumen 1125 and expiratory lumen 1135 are accessible from a left side of the patient interface.

With reference to Figure 5L, one end of the cannula manifold 1150 comprises a manifold inlet 1125a, for interfacing with or attaching to the inspiratory conduit 170 of the tube 174. With reference to Figure 5D, that end of the cannula manifold 1150 further comprises a manifold outlet 1135a, for interfacing with or attaching to the expiratory conduit 173 of the tube 174. The manifold inlet 1125a and the manifold outlet 1135a may be substantially parallel to each other. Alternatively, the manifold inlet 1125a and manifold outlet 1135a may both extend from one side of the patient interface, but at a non-parallel angle.

An opposite end 1151 of the cannula manifold 1150 is closed to form a plug or cap to close the first and second open ends 1111a, 1111b of the cannula body, depending on the insertion direction of the cannula manifold 1152.

A sealing surface 1151a, 1151b is provided at or towards each end of the cannula manifold 1150 to seal with the first and second open ends 1111a, 1111b of the body 1110 so that when the cannula manifold is inserted into the cavity 1111, the cannula manifold 1150 forms a seal with both the first and second open ends 1111a, 1111b.

The cannula manifold 1150 comprises an inspiratory gas delivery opening 1125b to communicate with the gas chamber in the cavity 1111 and one of the nasal elements when the cannula manifold 1150 is inserted into the cavity 1111.

The cannula manifold 1150 comprises an expiratory gases receipt opening 1135b that is substantially pneumatically isolated from the inspiratory gas delivery opening 1125b. The expiratory gases receipt opening 1135b communicates with the other one of the nasal elements when the cannula manifold 1150 is inserted into the cavity 1111.

With the cannula manifold 1150 inserted into the cavity 1111, the manifold inlet 1125a is in fluid communication with a section of the cavity 1111 via the inspiratory gas delivery opening 1125b. With the cannula manifold 1150 inserted into the cavity 1111, the manifold outlet 1135a is in fluid communication with another section of the cavity 1111 via the expiratory gases receipt opening 1135b.

In some embodiments, the body 1110 or the cannula manifold 1111 may comprise resilient material to form a seal between the cannula manifold 1111 part and the body 1110 to substantially prevent gas flow through the first and second open ends 1111a, 1111b between the body and the cannula manifold.

For example, the body 1110 may comprise a resilient material within the cavity 1111 to contact sealing surfaces 1151a, 1151b of the cannula manifold. In some embodiments, the resilient material may be integrally formed with a face contacting part of the 1110.

In some embodiments, the inspiratory gas delivery opening 1125b may be formed rearwardly, and in some embodiments may extend for a substantial length of the manifold. With the inspiratory gas delivery opening 1125b formed rearwardly, the resilient face contacting part of the frame may be unsupported by the manifold assembly in an upper lip (between upper lip and nose) area so that the face contacting part in the region of the inspiratory gas delivery opening 1125b provides a cushion to sit against the user's upper lip. The face contacting portion in the area of the manifold assembly outlet may act as an air-filled cushion to contact the user's upper lip region.

The manifold inlet 1125a and the inspiratory gas delivery opening 1125b form the manifold inspiratory lumen 1125 of the cannula manifold 1150. The manifold outlet 1135b and the expiratory gases receipt opening 1135b form the manifold expiratory lumen 1135 of the cannula manifold 1150.

The flow directing member 1152 is arranged such that a distal one of the first and second nasal elements relative to manifold inlet 1125a and the manifold outlet 1135a is an inspiratory nasal element for directing gas flow to the patient and a proximal one of the first and second nasal elements relative to the manifold inlet 1125a and the manifold outlet 1135a inspiratory lumen is an expiratory nasal element for directing a flow of expiratory gases away from the patient. When the cannula manifold 1150 is in the first configuration relative to the cannula body 1110, the first nasal element 1155a is the inspiratory nasal element and the second nasal element 1155b is the expiratory nasal element. When the cannula manifold 1150 is in the second configuration relative to the cannula body 1110, the second nasal element 1155b is the inspiratory nasal element and the first nasal element 1155a is the expiratory nasal element.

A proximal portion 1152a of the flow directing member 1152 extends substantially in a longitudinal direction of a body portion 1154 of the cannula manifold 1150. A distal portion 1152b of the of the flow directing member extends transversely from the distal end of the proximal portion 1152a to meet the body portion 1154 of the cannula manifold.

The distal portion 1152b may extend from the distal end of the proximal portion 1152a at a substantially perpendicular angle, or may extend from the distal end of the proximal portion 1152a at an obtuse angle.

A cavity defined between the proximal portion 1152a, the distal portion 1152b, and the body portion 1154 forms the expiratory gases receipt opening 1135b. The cavity that forms the expiratory gases receipt opening 1135b is in fluid communication with a proximal one of the nasal elements when the cannula manifold 1150 is in position in the cavity 1111 of the cannula body 1110, such that the nasal element expiratory lumen 1130 is in fluid communication with the cannula manifold expiratory lumen 1135.

The inspiratory gas delivery opening 1125b is provided on an opposite side of the distal portion 2152b of the flow directing member from the cavity.

A channel 1125c is provided on the opposite side of the proximal portion 1152a of the flow directing member and along a length of the proximal portion 1152a, and is in fluid communication with the manifold inlet 1125a and the inspiratory gas delivery opening 1125b. The inspiratory gas delivery opening 1125b is in fluid communication with a distal one of the nasal elements when the cannula manifold 1150 is in position in the cavity 1111 of the cannula body 1110, such that the nasal element inspiratory lumen 1210 is in fluid communication with the cannula manifold inspiratory lumen 1125.

In the configuration shown, the cannula manifold 1150 comprises an assembly of components.

In some embodiments, the cannula manifold 1150 comprises a first component 1150a and a second component 1150b engageable with the first component 1150a. The cannula manifold may comprise a third component 1150c that acts as a fastening component to hold the first and second components 1150a, 1150b together. The cannula manifold 1150 may be formed from a series of modular components that allow for relatively simple tool designs to be used to create the smooth transition between the manifold inlet 1125a and the inspiratory gas delivery opening 1125b of the cannula manifold. In particular, using a modular design allows a combination of relatively simple components to be assembled together to form a more complex shape. Alternatively, the cannula manifold may have fewer components.

An inner end of the second component 1150b be received into a recess of the first component 1150a. The third component 1150c may comprise an annular sleeve that is configured to receive outer ends of the first component 1150a and the second component 1150b to hold the outer ends together. The first component 1150a and second component 1150b and the third component 1150c may have suitable engagement features which maintain the components in engagement with each other, such as clips, fasteners, or any other suitable feature.

The cannula manifold 1152 may comprise a rigid polymer component. The cannula manifold 1152 may comprise polycarbonate for example.

It will be appreciated that the configuration of the cannula manifold 1152 including the flow directing member 1152 may vary depending on the cannula that the cannula manifold 1152 will be used with.

The patient interface 1100 may be provided in combination with, or may be integrally formed with, any of the tube 174 configurations described above.

For example, the patient interface 1110 may comprise or be coupled to the inspiratory conduit 170 that defines or is in fluid communication with the inspiratory lumen 1125, and may comprise or be coupled to the expiratory conduit 173 that defines or is in fluid communication with the expiratory lumen 1135.

The inspiratory conduit 170 may be coupled to manifold inlet 1125a. The expiratory conduit 173 may be coupled to manifold outlet 1135a.

The inspiratory conduit 170 and the expiratory conduit 173 may be coaxial, substantially parallel, or non-parallel.

The inspiratory conduit 170 and the expiratory conduit 173 may be on a single limb, such that the inspiratory element and the expiratory element both extend in one direction from the cannula body 1110 in use.

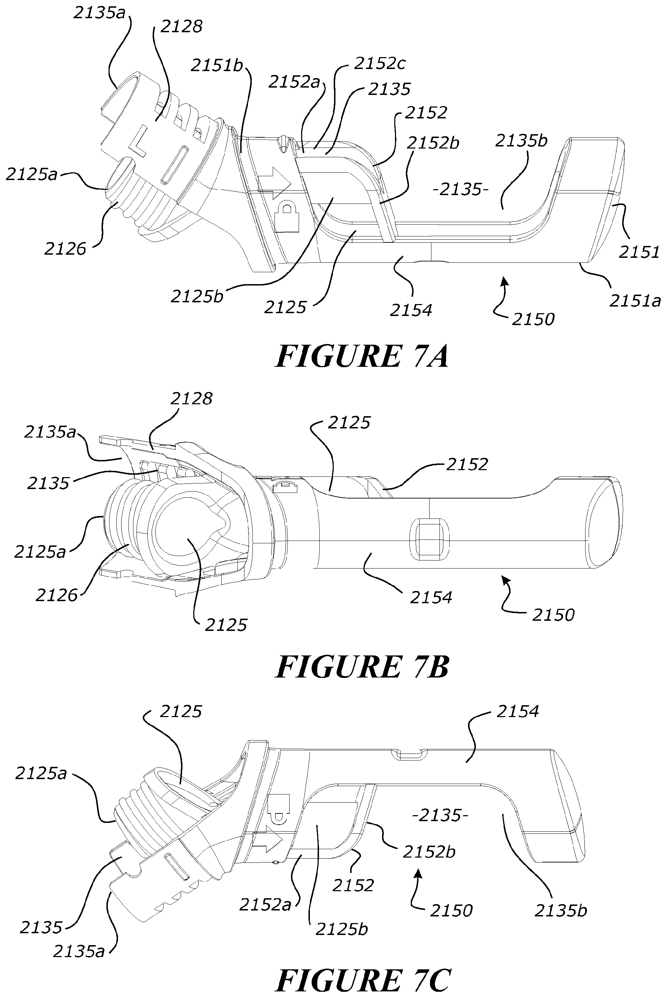

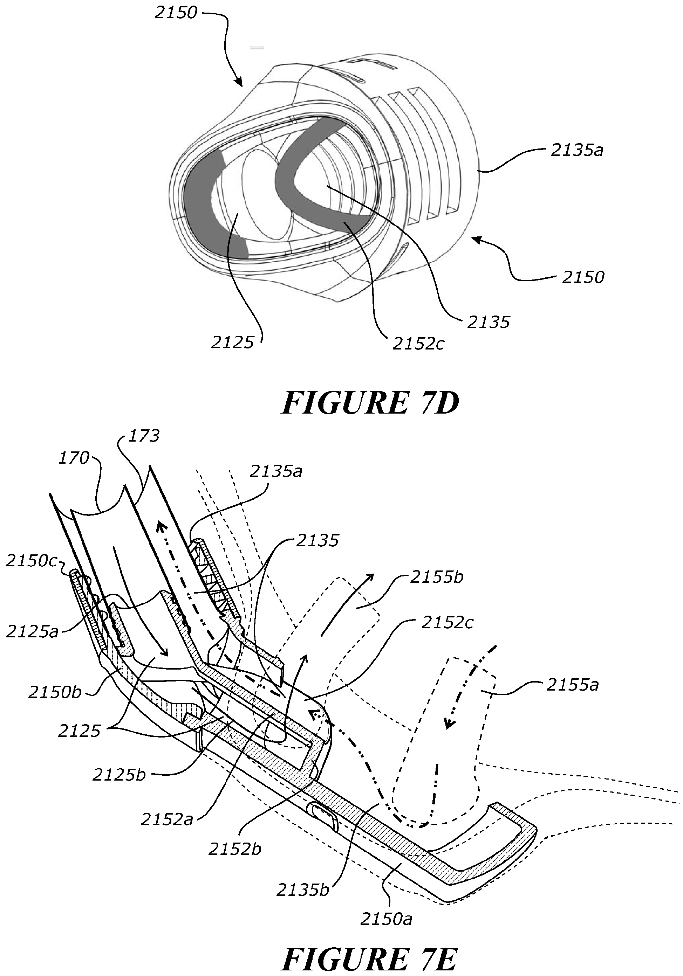

Figures 7A to 7E show an alternative configuration cannula manifold 2150 and Figures 6C and 6D schematically such flow paths through that cannula manifold 2150. Unless described differently below, the features, functionality, and options are the same as for the cannula manifold 1150, and like reference numerals indicate like parts with the addition of 1000.

In this configuration of the cannula manifold 2150, the flow directing member 2152 is arranged such that a proximal one of the first and second nasal elements relative to the manifold inlet 2125a and the manifold outlet 2135a is an inspiratory nasal element for directing gas flow to the patient and a distal one of the first and second nasal elements relative to the manifold inlet 2125a and the manifold outlet 2135a is an expiratory nasal element for directing a flow of expiratory gases away from the patient.

In the first configuration of the cannula manifold 2150 relative to the cannula body 1110 shown in Figure 6C, the right nasal element 1155b (relative to the patient) is the inspiratory nasal element and the left nasal element 1155a is the expiratory nasal element. In the second configuration of the cannula manifold 2150 relative to the cannula body 1110 shown in Figure 6D, the left nasal element 1155a is the inspiratory nasal element in the right nasal element 1155b is the expiratory nasal element.

A proximal portion 2152a of the flow directing member 2152 extends substantially in a longitudinal direction of a body portion 2154 of the cannula manifold 2150. A distal portion 2152b of the of the flow directing member extends transversely from the distal end of the proximal portion 2152a to meet the body portion 2154 of the cannula manifold.

The distal portion 1152b may extend from the distal end of the proximal portion 1152a at a substantially perpendicular angle, or may extend from the distal end of the proximal portion 1152a at an obtuse angle.

A cavity defined between the proximal portion 2152a, the distal portion 2152b, and the body portion 2154 forms the inspiratory gas delivery opening 2125b that is in fluid communication with the manifold inlet 2125a. The cavity that forms the inspiratory gas delivery opening 2125b is in fluid communication with the proximal one of the nasal elements when the cannula manifold 1150 is in position in the cavity 1111 of the cannula body 1110, such that the nasal element inspiratory lumen 1120 is in fluid communication with the cannula manifold inspiratory lumen 2125.

The expiratory gases receipt opening 2135b is provided on an opposite side of the distal portion 2152b of the flow directing member from the cavity.

A channel 2152c is provided on the opposite side of the proximal portion 2152a of the flow directing member, and is in fluid communication with the manifold outlet 2135a and expiratory gases receipt opening 2135b. The expiratory gases receipt opening 2135b is in fluid communication with the distal one of the nasal elements when the cannula manifold 2150 is in position in the cavity 1111 of the cannula body 1110, such that the nasal element expiratory lumen 1130 in fluid communication with the cannula manifold expiratory lumen 2125.

The inner tubular component 2126 that provides the manifold inlet 2125a and the outer tubular component that provides the manifold outlet 2135a are coaxial. The patient interface 1100 with this alternative cannula manifold 2150 may be provided in combination with, or may be integrally formed with, any of the coaxial tube 174 configurations described above. The coaxial configuration used with the coaxial tube 174 reduces the number of separate conduits proximal to the patient.

The components may be configured with any suitable dimensions, depending on the desired applications and properties. For example, the inspiratory limb inner tubular component 2126 may have an inner hydraulic diameter range of between about 3 mm and about 13 mm, and the expiratory limb outer tubular component 2128 may have an outer hydraulic diameter of between about 10 mm and about 30 mm, and in some configurations up to about 23 mm. The geometry may be selected to provide a desired resistance to flow. For example, the components may be circular/cylindrical or any suitable non-circular shapes.

The cannula manifold 2150 may comprise second and third components similar to second and third components 1125b, 1125c of cannula manifold 1150.

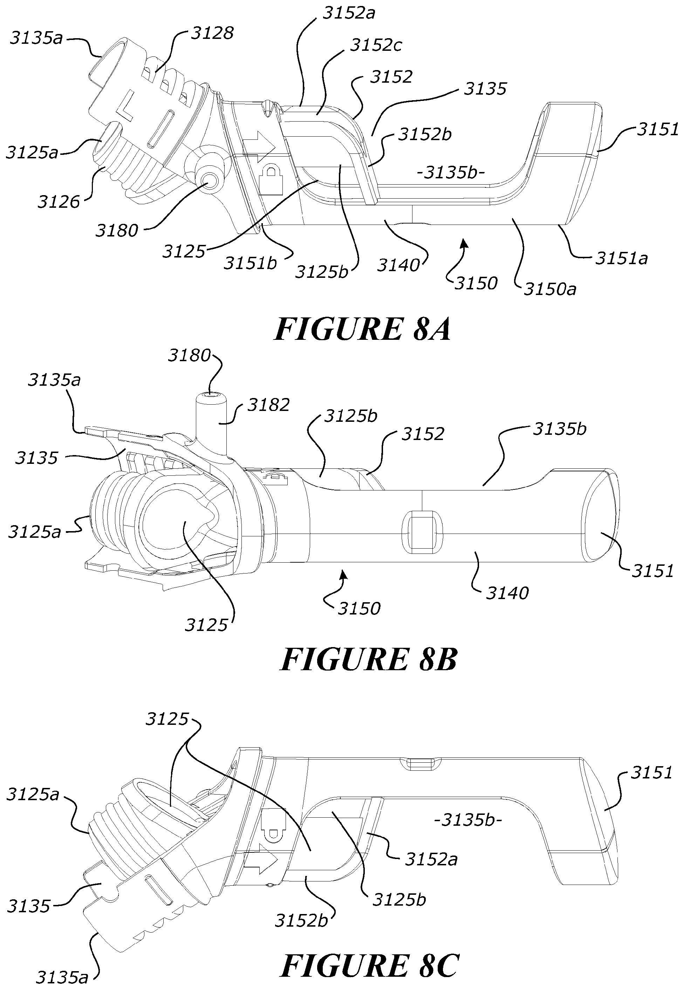

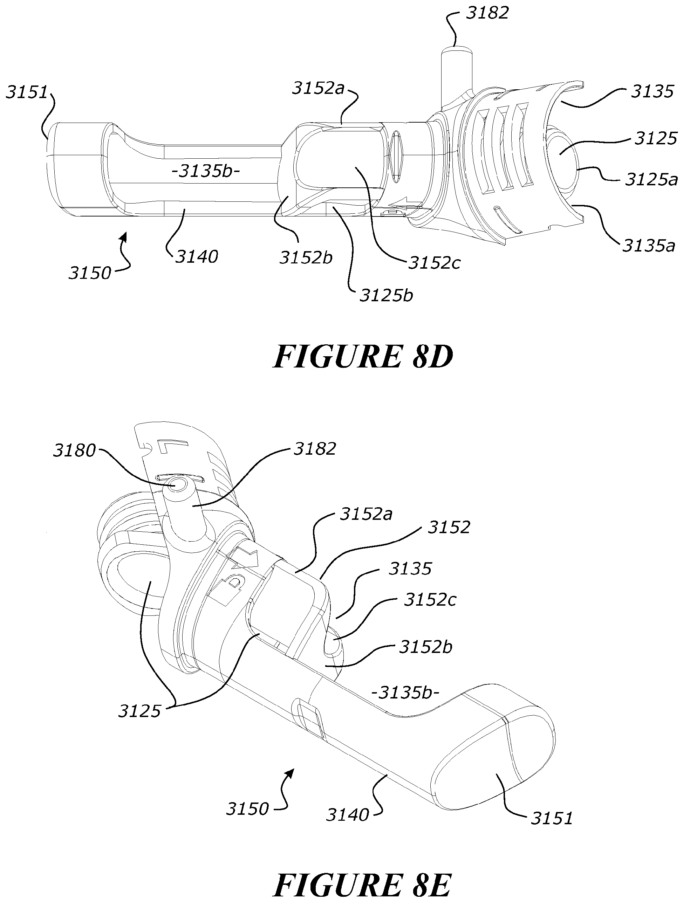

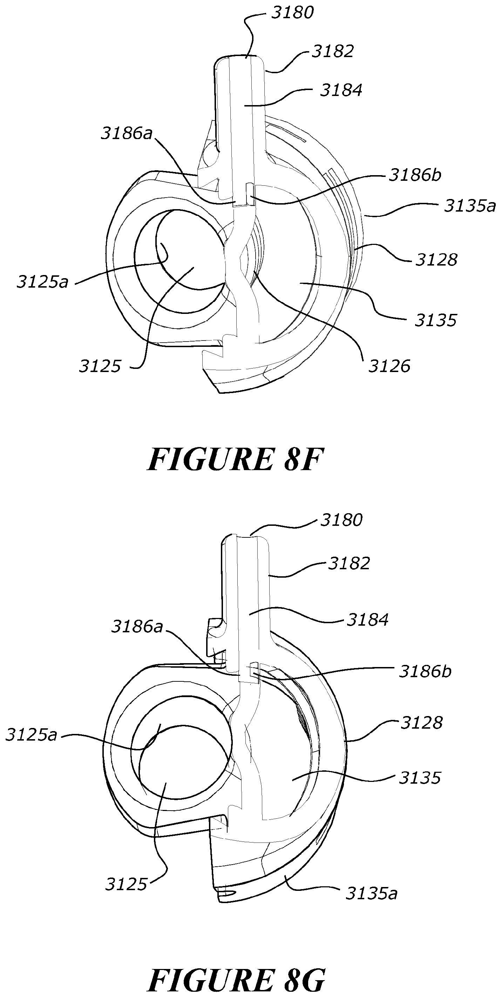

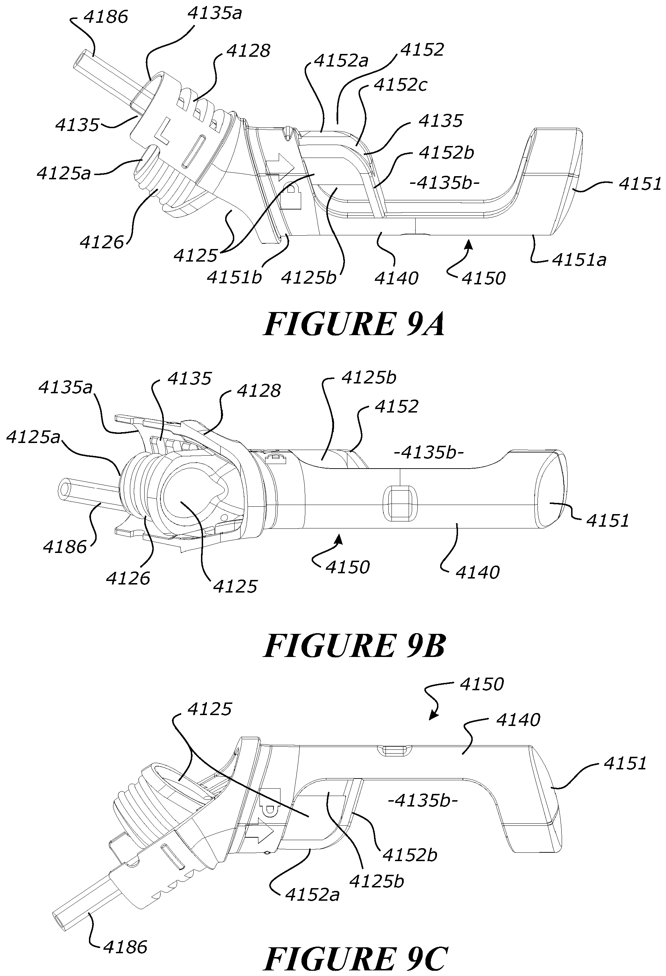

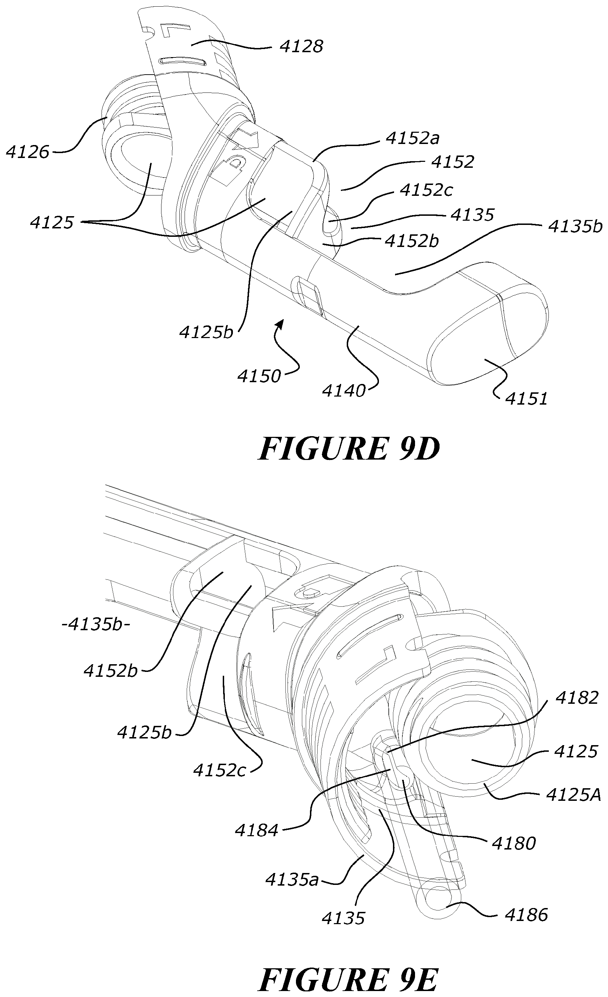

The cannula manifolds 1150, 2150 described herein may be provided with a pressure monitoring port. Figures 8A to 8G and 9A to 9F show two alternative cannula manifolds 3150, 4150 with pressure monitoring ports. Unless described below, the features, functionality, and options are the same as for cannula manifold 2150, and like reference numbers are used to indicate like parts with the addition of 1000 and 2000 respectively.

With reference to Figures 8A to 8G, the cannula manifold 3150 comprises a manifold inspiratory lumen 3125 for inspiratory gas flow and a manifold expiratory lumen 3135 for expiratory gas flow.

The cannula manifold 3150 comprises a pressure monitoring port 3180. The pressure monitoring port 3180 is in fluid communication with the manifold inspiratory lumen 3125 and the manifold expiratory lumen 3135.

The pressure monitoring port 3180 is configured to provide a gas flow for pressure monitoring through the pressure monitoring port, the gas flow comprising an average of the inspiratory gas flow and expiratory gas flow. The averaging of the inspiratory gas flow and expiratory gas flow is applicable to a unidirectional flow system.

In the configuration shown, the pressure monitoring port 3180 is provided in a tubular member 3182 that projects outwardly from the body 3140 of the cannula manifold 3150.

A lumen 3184 extends from a base of the tubular member 3182 and terminates at the pressure monitoring port 3180 at an outer end of the tubular member 3180.

The tubular member 3182 is provided in the section of the cannula manifold corresponding to the coaxial members 3126, 3128 that provide the manifold inlet 3125a and the manifold outlet 3135a.

A base of the tubular member 3184 comprises an inspiratory gases port 3186a in fluid communication with the manifold inspiratory lumen 3125 and an expiratory gases port 3186b in fluid communication with the manifold expiratory lumen 3135. The lumen 3184 in the tubular member provides fluid communication between the inspiratory gases port 3186a, the expiratory gases port 3186b, and the pressure monitoring port 3180.

When gases pass through the manifold inspiratory lumen 3125 and the manifold expiratory lumen 3135, some of the inspiratory gases and expiratory gases will be able to pass through the ports 3186a, 3186b, and the pressure monitoring port 3180.

The pressure monitoring port 3180 is fluidly connected to both nasal elements via the manifold inspiratory lumen 3125 and the manifold expiratory lumen 3135. The pressure monitoring port 3180 may have a high enough resistance to flow to minimise gases flow that escapes through the pressure monitoring port 3180. However, the dimensions can be selected to minimise not only gases flow into the pressure monitoring port 3180 but also between the manifold inspiratory lumen 3125 and the manifold expiratory lumen 3135 which could otherwise decrease the effects induced by the unidirectional flow design as described herein. Pressure measurement of the manifold inspiratory lumen 3125 and the manifold expiratory lumen 3135, effectively averaging the inspiratory and expiratory pressures, is useful as a good estimate of the naso-pharyngeal pressure inside the patient. A traditional pressure measurement tube that is fluidly connected to an inspiratory or expiratory circuit component can only measure the inspiratory or expiratory pressure, respectively, which is not necessarily a good estimate of the naso-pharyngeal pressure.

Advantageously, the pressure monitoring port 3180 provides the ability to measure or determine a patient pressure, without the need to position or put a sensor inside the patient's airway.

If the patient interface and cannula manifold 3150 are symmetrical or substantially symmetrical, then the measured pressure is provided as a useful approximation of the patient pressure.

Alternatively, if the patient interface and cannula manifold 3150 has a predictable, known or predetermined asymmetry, then the measured pressure plus or minus an offset to account for the asymmetry will result in another useful approximation of the patient pressure. The specific amount of the offset may be dependent on, for example, the geometry of the patient interface and is calculable by one of skill in the art using known methodology.

With reference to Figures 9A to 9F, an alternative cannula manifold 4150 comprises a manifold inspiratory lumen 4125 for inspiratory gas flow and a manifold expiratory lumen 4135 for expiratory gas flow.

The cannula manifold 4150 comprises a pressure monitoring port 4180. The pressure monitoring port 4180 is in fluid communication with the manifold inspiratory lumen 4125 and the manifold expiratory lumen 4135.

The pressure monitoring port 4180 is configured to provide a gas flow for pressure monitoring through the pressure monitoring port, the gas flow comprising an average of the inspiratory gas flow and expiratory gas flow.

In the configuration shown, the pressure monitoring port 4180 is provided in a tubular member 4182 that projects in an axial direction between the inspiratory limb inner tubular component 4126 and the expiratory limb outer tubular component 4128.

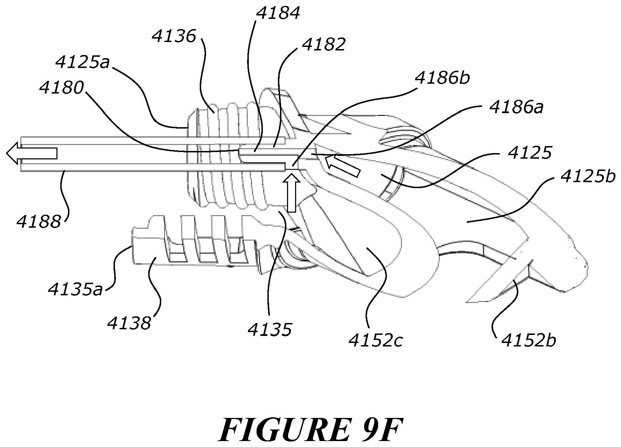

With reference to Figure 9F, a lumen 4184 extends from a base of the tubular member 4182 and terminates at the pressure monitoring port 4180 at an outer end of the tubular member 4180.

A base of the tubular member 4184 comprises an inspiratory gases port 4186a in fluid communication with the manifold inspiratory lumen 4125 and an expiratory gases port 4186b in fluid communication with the manifold expiratory lumen 4135. The lumen 4184 in the tubular member provides fluid communication between the inspiratory gases port 4186a, the expiratory gases port 4186b, and the pressure monitoring port 4180.

When gases pass through the manifold inspiratory lumen 4125 and the manifold expiratory lumen 4135, some of the inspiratory gases and expiratory gases will be able to pass through the ports 4186a, 4186b, and the pressure monitoring port 4180.

The pressure monitoring port 3180, 4180 of the cannula manifold 3150, 4150 may be in fluid communication with any suitable pressure monitoring device, such as a pressure sensor. An extension tube 4186 may be coupled to the pressure monitoring port 3180, 4180 to couple that to the pressure monitoring device.

Although the pressure monitoring ports 3180, 4180 are described with reference to cannula manifolds 3150, 4150 with coaxial manifold inlets 3125a, 4125a and manifold outlets 3135a, 4135a, the pressure monitoring ports may be provided in cannula manifolds that have a non-coaxial arrangement of manifold inlets and manifold outlets. Additionally, or alternatively, the pressure monitoring ports could be provided in cannula manifolds that deliver gases to the distal nasal element and that receive expiratory gases from the proximal nasal element, rather than the opposite configuration shown.

Any of the patient interfaces 1100 and/or cannula manifolds 1150, 2150, 3150, 4150 may comprise a filter 140 in fluid communication with the patient interface or manifold expiratory lumen 1135, 2135, 3135, 4135 (e.g. via manifold outlet 1135a, 2135a, 3135a, 4135a), the filter 140 configured to filter the flow of gas from the patent interface before passage of the flow of expiratory gases from the patient interface into ambient air.

The filter 140 and/or tube 174 to couple the filter to the patient interface 1100 or cannula manifold 1150, 2150, 3150, 4150 may have any of the features described or shown herein.

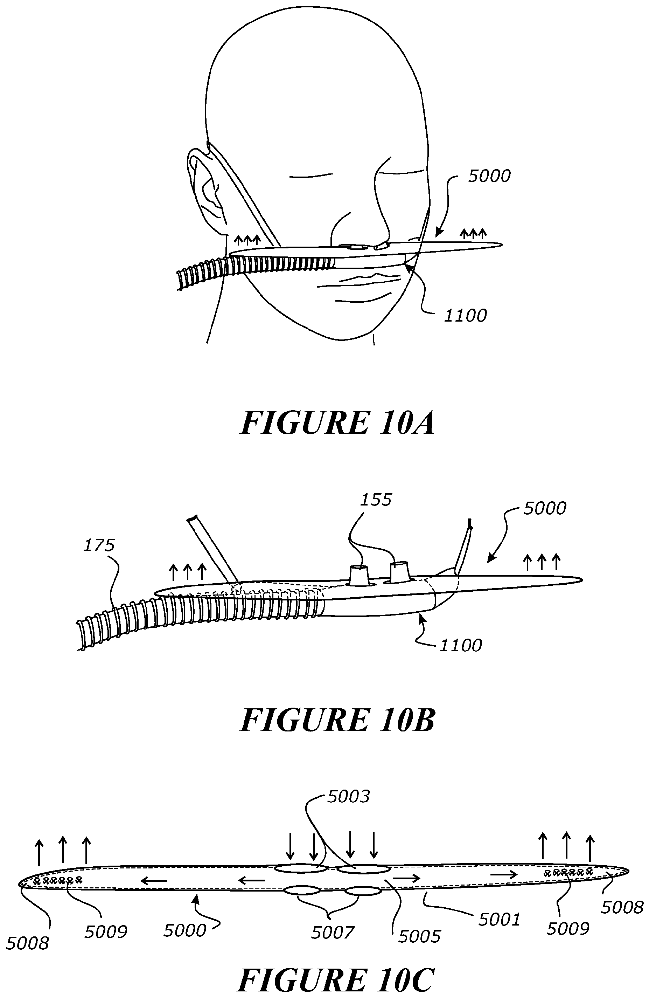

Figures 10A-10E show an external flow director on a cannula body of the patient interface for capturing and redirecting at least a portion of expiratory flow to divert and/or slow down exhaled gases flow. The external flow director can be provided as part of, or used in combination with, any of the patient interfaces 1100 disclosed herein.

Aerosols that are directed forwards with high velocity from an infectious patient are thought to be more likely of coming into contact with others such as hospital staff and visitors. Flow that is directed downwards, upwards, sideways or behind the patient is thought to have a lower likelihood of infecting other people. Similarly, flow that has a lower velocity is not able to carry infectious aerosols as far, and thus also reduces infection risk. The following concepts are thus designed to alter the direction and/or velocity of expiratory flow from a patient that is receiving therapy, such as through a high-flow nasal cannula or other patient interface for example.

In some configurations, the patient interface 1100 comprises an external flow director 5000 to divert and/or slow down exhaled gases flow. The external flow director 5000 may comprise one or more structures or modifications to the cannula body (i.e. may be integrally formed with the cannula body) or may comprise one or more attachments that can be optionally used with the patient interface if and when required. That is, the external flow director 5000 may comprise cannula body feature(s) or attachment(s).

The external flow director 5000 may encourage exhaled gases to travel outwardly away from the patient in more desirable directions, i.e. in one or more directions that may assist to reduce risk of transmission of diseases or undesirable substances from patient expired gases.

As well as providing an arrangement for diverting expiratory gases flow in a more desirable direction, the external flow director 5000 can alternatively or additionally direct the captured expiratory gases flow to filter, such as a filter tube of the type described above.

Figure 10A shows an exemplary patient interface 1100 in use on a patient. The patient interface 1100 comprises a cannula body with an external flow director 5000. Figures 10B and 10C show more detail of the external flow director 5000.

The external flow director 5000 is configured to capture and redirect expiratory flow.

The external flow director 5000 comprises an elongated, tubular, rigid body 5001. The body 5001 may sit or be supported on top of the cannula body. The nasal elements 155 extend up through the external flow director 5000 to enter the nares of patient.

Both nasal elements 155 may deliver flow. Both nasal elements 155 may be non-sealing.

Expiratory gases exiting the nares are able to enter the external flow director 5000 through apertures 5003 in the external flow director around each nasal element 155. The size of these apertures 5003 are larger than the size of the portions of the nasal elements 155 that extend through the apertures 5003. The size of these apertures 5003 may be of the same approximate size as the patient's nares, or they may be larger than the nares for a larger capture area.

The upper surface of the external flow director 5000, which comprises the apertures 5003, will be displaced from the nares by a small distance in use. In other words, the apertures 5003 are non-sealing. In another embodiment, the upper surface of the external flow director 5000, may be configured to seal with the nares.

A mesh or filter may cover the apertures 5003 to diffuse and/or filter flow as it enters the internal chamber 5005 of the cannula body feature or attachment 5000.

The external flow director 5000 comprises two further lower apertures 5007 on the bottom of the external flow director 5000, through which the nasal elements are inserted when the cannula body feature or attachment 5000 is attached to the cannula. These apertures 5007 will substantially seal with the cannula such that expiratory gases cannot escape the attachment through the lower apertures 5007. Alternatively, the cannula body feature or attachment 5000 may be integrally formed with the cannula body 174 such that it is not removable.

The external flow director 5000 is configured such that gases flow that enters the external flow director 5000 is then redirected along its length. In one configuration, one or both ends 5008 of the external flow director 5000 that are distal from the nasal elements 155 may comprise one or a plurality of small apertures 5009 that direct the flow upwards, backwards, downwards, sideways, or some combination thereof.

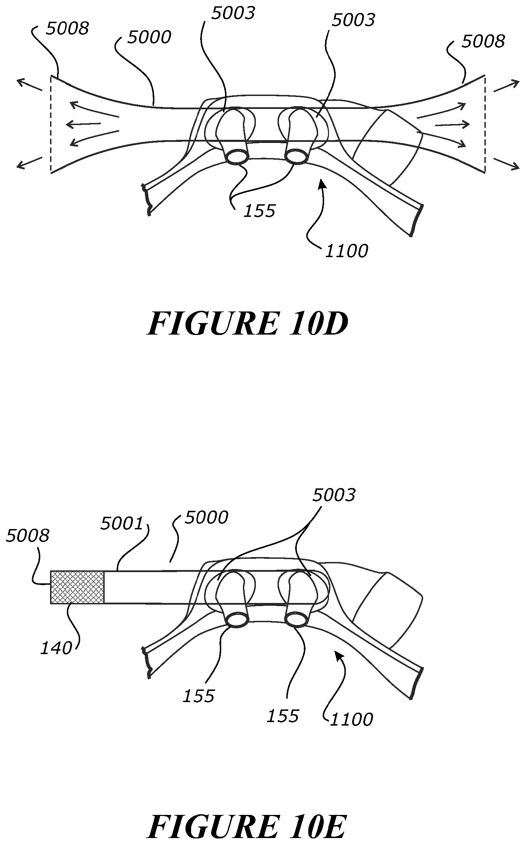

In an alternative configuration shown in Figure 10D, one or both ends 5008 of the external flow director 5000 may be open, to direct the gases flow in a lateral direction away from the patient.

The body 5001 of the external flow director 5000 may have a cross-sectional area that enlarges towards to end(s) 5008. That will have the effect of decreasing the velocity of the gases flow out of the open ends.

In an alternative configuration shown in Figure 10E, the external flow director 5000 may only extend from the nasal elements 155 laterally in a single direction.

The distal end 5008 of the external flow director 5000 may comprise a filter and/or diffuser 140 that is configured to capture aerosols and/or decrease the velocity of gases flow exiting the distal end 5008 of the external flow director 5000.

The filter and/or diffuser 140 may be provided in one or more walls of the body 5001, or may be provided by a separate filter component that provides the filter.

Embodiments of the present disclosure have provided examples of various patient interfaces using gas permeable bodies and/or physical structures to treat expiratory gases before exit from the breathing circuit. These may comprise part of or the entirety of the novel patient interface of the present disclosure, or they may be provided as part of an accessory to be fitted or applied to existing patient interfaces such as nasal cannulas, masks, ETTs, tracheostomy tubes or the like.

Embodiments of the disclosure are directed, although not limited, to improvements in patient interfaces for delivering respiratory therapy to patients, which improvements may be targeted at reducing risk of infection of staff and others during treatment of infective patients expiring airborne pathogens. For example, in the case of SARS-CoV-2, respiratory droplets are known to carry a transmission risk. However, the risk of transmission by airborne droplets is not well understood. The present disclosure provides, at the very least, a precautionary improvement to patient interfaces that may be used in the delivery of NHF and other respiratory therapies (including positive pressure therapies) that may cause aerosolization of particles in expiratory gases particularly for patients known to have infective or transmissible respiratory illness.

The patient interfaces, cannula manifolds, and/or tubes and conduits may have any one or more of the features described in PCT/IB2015/054585 (published as WO 2015/193833) and US patent no. 10,806,887. The contents of those specifications are incorporated herein in their entirety by way of reference.

It is to be understood that various modifications, additions and/or alterations may be made to the parts previously described without departing from the ambit of the present invention as defined in the claims appended hereto.

Future patent applications may be filed in the United States of America or overseas on the basis of or claiming priority from the present application. It is to be understood that the following claims are provided by way of example only, and are not intended to limit the scope of what may be claimed in any such future application. Features may be added to or omitted from the claims at a later date so as to further define or re-define the invention or inventions.
This divisional application is divided from EP21909679.9 (the 'parent application') and the divisional specification as filed comprises the content of the parent application, including the original claims recited as 'representative features' below. The scope of this disclosure therefore includes the full content of the parent application. In any case, protection may be sought for any features disclosed in the parent application as filed.

### REPRESENTATIVE FEATURES

1. A tube for delivery of gases to a patient interface and for treatment of expiratory gases, the tube comprising:
   an inspiratory conduit for directing a flow of gas toward a patient interface;
   an expiratory conduit for directing a flow of expiratory gases from the patient interface;
   and a filter in fluid communication with, or part of, the expiratory conduit, the filter configured to filter the flow of expiratory gases from the patent interface before passage of the flow of expiratory gases from the patient interface into ambient air.
2. The tube according to clause 1, wherein the inspiratory conduit and the expiratory conduit are coaxial or substantially parallel.
3. The tube according to clause 2, wherein the inspiratory conduit and the expiratory conduit are on a single limb, such that the inspiratory conduit and the expiratory conduit both extend in one direction from the patient interface in use.
4. The tube according to clause 3, wherein the inspiratory conduit is an inner conduit, and wherein the expiratory conduit is an outer conduit.
5. The tube according to any one of clauses 1 to 4, wherein a wall of the expiratory conduit comprises the filter.
6. The tube according to clause 5, wherein the filter comprises one or more filter materials selected from the group comprising: an N95 or higher (e.g. N99) rated or HEPA filter material, fibrous, porous knitted, mesh, woven foam, textile, non-textile, or foam filter material.
7. The tube according to clause 5 or 6, wherein a length of the filter in the wall is configured to reduce a resistance to flow of gases through the filter.
8. The tube according to any one of clauses 5 to 7, wherein a portion of the expiratory conduit upstream of the filter has a wall that is substantially non-permeable.
9. The tube according to clause 8, wherein the portion of the expiratory conduit upstream of the filter has a length of at least about 80 mm.
10. The tube according to any one of clauses 1 to 4, wherein the expiratory conduit is coupled to a filter component that comprises the filter.
11. The tube according to clause 10, wherein the filter comprises one or more filter materials selected from the group comprising: an N95 or higher (e.g. N99) rated or HEPA filter material, fibrous, porous knitted, mesh, woven foam, textile, non-textile, or foam filter material.
12. The tube according to any one of clauses 1 to 11, wherein the expiratory conduit comprises structure to prevent or minimise collapse or constriction of an expiratory lumen in the expiratory conduit.
13. The tube according to clause 12, wherein the structure comprises one more of: corrugations, pleats, helical wrap, ribs, supporting features.
14. The tube according to any one of clauses 1 to 13, wherein the inspiratory conduit is configured to direct a flow of gas toward at least one inspiratory lumen of the patient interface, and wherein the expiratory conduit is configured to direct a flow of gas from at least one expiratory lumen of the patient interface.
15. The combination of a patient interface and the tube of clause 14, wherein the patient interface comprises: at least one inspiratory element for directing a flow of gas to a patient airway, the or each inspiratory element comprising the at least one inspiratory lumen through which said flow of gas is directed; and at least one expiratory element comprising the expiratory lumen that provides an expiratory gas flow path.
16. The combination according to clause 15, wherein the patient interface comprises first and second nasal elements configured to direct gas into and/or from a nare of the patient.
17. The combination according to clause 16, wherein the at least one inspiratory element comprises one or both of the first and second nasal elements, and wherein the at least one expiratory element comprises one or both of the first and second nasal elements.
18. The combination according to clause 16 or 17, wherein the first nasal element comprises a first nasal prong and wherein the second nasal element comprises a second nasal prong.
19. The combination according to any one of clauses 16 to 18, wherein the patient interface comprises a flow directing member to direct gas flow from the inspiratory conduit to one of the first and second nasal elements, and to direct gas flow from the other of the first and second nasal elements to the expiratory conduit.
20. The combination according to any one of clauses 16 to 18, wherein the patient interface comprises a partition or other physical separation to provide for a mechanical fluid disconnection between a fluid flow path of the first nasal element and a fluid flow path of the second nasal element.
21. The combination according to clause 19 or 20, comprising a gases port for entrainment of expiratory gases from a patient's nose and/or a patient's mouth that are not directed into an expiratory nasal element.
22. An expiratory tube for treatment of expiratory gases, the expiratory tube comprising:
   an expiratory conduit for directing a flow of expiratory gases from a patient interface;
   and a filter in fluid communication with, or part of, the expiratory conduit, the filter configured to filter the flow of expiratory gases from the patent interface before passage of the flow of expiratory gases from the patient interface into ambient air.
23. The expiratory tube according to clause 22, wherein a wall of the expiratory conduit comprises the filter.
24. The expiratory tube according to clause 23, wherein the filter comprises one or more filter materials selected from the group comprising: an N95 or higher (e.g. N99) rated or HEPA filter material, fibrous, porous knitted, mesh, woven foam, textile, non-textile, or foam filter material.
25. The expiratory tube according to clause 22 or 23, wherein a length of the filter in the wall is configured to reduce a resistance to flow of gases through the filter.
26. The expiratory tube according to any one of clauses 22 to 25, wherein a portion of the expiratory conduit upstream of the filter has a wall that is substantially non-permeable.
27. The expiratory tube according to clause 26, wherein the portion of the expiratory conduit upstream of the filter has a length of at least about 80 mm.
28. The expiratory tube according to clause 22, wherein the expiratory conduit is coupled to a filter component that comprises the filter.
29. The expiratory tube according to clause 28, wherein the filter comprises one or more filter materials selected from the group comprising: an N95 or higher (e.g. N99) rated or HEPA filter material, fibrous, porous knitted, mesh, woven foam, textile, non-textile, or foam filter material.
30. The expiratory tube according to any one of clauses 22 to 29, wherein the expiratory conduit comprises structure to prevent or minimise collapse or constriction of an expiratory lumen in the expiratory conduit.
31. The expiratory tube according to clause 30, wherein the structure comprises one more of: corrugations, pleats, helical wrap, ribs, supporting features.
32. The expiratory tube according to any one of clauses 22 to 31, wherein the expiratory conduit is configured to direct a flow of gas from at least one expiratory lumen of the patient interface.
33. The combination of a patient interface and the expiratory tube of any one of clauses 22 to 32, wherein the patient interface comprises: at least one inspiratory element for directing a flow of gas to a patient airway, the or each inspiratory element comprising the at least one inspiratory lumen through which said flow of gas is directed; and at least one expiratory element comprising the expiratory lumen that provides an expiratory gas flow path.
34. The combination according to clause 33, wherein the patient interface comprises first and second nasal elements configured to direct gas into and/or from a nare of the patient.
35. The combination according to clause 34, wherein the at least one inspiratory element comprises one or both of the first and second nasal elements, and wherein the at least one expiratory element comprises one or both of the first and second nasal elements.
36. The combination according to clause 34 or 35, wherein the first nasal element comprises a first nasal prong and wherein the second nasal element comprises a second nasal prong.
37. A patient interface for delivery of gas to a patient, the patient interface comprising:
   at least one inspiratory lumen for directing a flow of gas to a patient airway;
   at least one expiratory lumen comprising an expiratory gas flow path for directing a flow of expiratory gases from the patient;
      and
   a filter in fluid communication with, or part of, the expiratory gas flow path, the filter configured to filter the flow of expiratory gases from the patent interface before passage of the flow of expiratory gases from the patient interface into ambient air.
38. The patient interface according to clause 37, comprising an inspiratory conduit that is in fluid communication with the inspiratory lumen, and an expiratory conduit that is in fluid communication with the expiratory lumen.
39. The patient interface according to clause 38, wherein the inspiratory conduit and the expiratory conduit are integrally formed with the patient interface or are coupled to the patient interface.
40. The patient interface according to clause 38 or 39, wherein the inspiratory conduit and the expiratory conduit are coaxial or substantially parallel.
41. The patient interface according to clause 40, wherein the inspiratory conduit and the expiratory conduit are on a single limb, such that the inspiratory conduit and the expiratory conduit both extend in one direction from the patient interface.
42. The patient interface according to clause 40 or 41, wherein the inspiratory conduit is an inner conduit, and wherein the expiratory conduit is an outer conduit.
43. The patient interface according to any one of clauses 37 to 42, wherein a wall of the expiratory conduit comprises the filter.
44. The patient interface according to clause 43, wherein the filter comprises one or more filter materials selected from the group comprising: an N95 or higher (e.g. N99) rated or HEPA filter material, fibrous, porous knitted, mesh, woven foam, textile, non-textile, or foam filter material.
45. The patient interface according to clause 43 or 44, wherein a length of the filter in the wall is configured to reduce a resistance to flow of gases through the filter.
46. The patient interface according to any one of clauses 43 to 45, wherein a portion of the expiratory conduit upstream of the filter has a wall that is substantially non-permeable.
47. The patient interface according to clause 46, wherein the portion of the expiratory conduit upstream of the filter has a length of at least about 80 mm.
48. The patient interface according to any one of clauses 37 to 42, wherein the expiratory conduit is coupled to a filter component that comprises the filter.
49. The patient interface according to clause 48, wherein the filter comprises one or more filter materials selected from the group comprising: an N95 or higher (e.g. N99) rated or HEPA filter material, fibrous, porous knitted, mesh, woven foam, textile, non-textile, or foam filter material.
50. The patient interface according to any one of clauses 37 to 49, wherein the expiratory conduit comprises structure to prevent or minimise collapse or constriction of an expiratory lumen in the expiratory conduit.
51. The patient interface according to clause 50, wherein the structure comprises one more of: corrugations, pleats, helical wrap, ribs, supporting features.
52. The patient interface according to any one of clauses 37 to 51, wherein the patient interface comprises first and second nasal elements configured to direct gas into and/or from a nare of the patient.
53. The patient interface according to clause 52, wherein the first nasal element comprises a first nasal prong, and wherein the second nasal element comprises a second nasal prong.
54. The patient interface according to clause 52 or 53, comprising a flow directing member to direct gas flow to one of the first and second nasal elements, and to direct gas flow from the other of the first and second nasal elements.
55. The patient interface according to clause 52 or 53, wherein the patient interface comprises a partition or other physical separation to provide for a mechanical fluid disconnection between a fluid flow path of the first nasal element and a fluid flow path of the second nasal element.
56. The patient interface according to clause 54 or 55, comprising a gases port for entrainment of expiratory gases from a patient's nose and/or a patient's mouth that are not directed into an expiratory nasal element.
57. A patient interface for respiratory therapy comprising:
   a cannula body comprising a first nasal element and a second nasal element; and
   a cannula manifold, the cannula manifold comprising a manifold inspiratory lumen, a manifold expiratory lumen, and a flow directing member, the cannula manifold being reconfigurable relative to the cannula body such that in a first configuration the flow directing member is arranged to direct gas flow from the manifold inspiratory lumen to the first nasal element and to direct gas flow from the second nasal element to the manifold expiratory lumen, and in a second configuration the flow directing member is arranged to direct gas flow from the manifold inspiratory lumen to the second nasal element and to direct gas flow from the first nasal element to the manifold expiratory lumen.
58. The patient interface according to clause 57, wherein the cannula manifold is configured for insertion into the cannula body.
59. The patient interface according to clause 57 or 58, comprising or coupled to an inspiratory conduit that is in fluid communication with the inspiratory lumen, and comprising or coupled to an expiratory conduit that in fluid communication with the expiratory lumen.
60. The patient interface according to clause 58 or 59, wherein the inspiratory conduit and the expiratory conduit are coaxial or substantially parallel.
61. The patient interface according to clause 60, wherein the inspiratory conduit and the expiratory conduit are on a single limb, such that the inspiratory element and the expiratory element both extend in one direction from the cannula body.
62. The patient interface according to clause 57, wherein the inspiratory conduit is an inner conduit, and wherein the expiratory conduit is an outer conduit.
63. The patient interface according to any one of clauses 57 to 62, wherein the flow directing member is arranged such that a distal one of the first and second nasal elements relative to a manifold inlet and a manifold outlet is an inspiratory nasal element for directing gas flow to the patient and a proximal one of the first and second nasal elements relative to the manifold inlet and the manifold outlet is an expiratory nasal element for directing a flow of expiratory gases away from the patient.
64. The patient interface according to any one of clauses 57 to 62, wherein the flow directing member is arranged such that a proximal one of the first and second nasal elements relative to a manifold inlet and a manifold outlet is an inspiratory nasal element for directing gas flow to the patient and a distal one of the first and second nasal elements relative to the manifold inlet and the manifold outlet is an expiratory nasal element for directing a flow of expiratory gases away from the patient.
65. The patient interface according to clause 63 or 64, comprising a gases port for entrainment of expiratory gases from a patient's nose and/or a patient's mouth that are not directed into the expiratory nasal element.
66. The patient interface according to any one of clauses 57 to 65, wherein the cannula manifold comprises a body portion, and wherein the flow directing member comprises a proximal portion that extends substantially in a longitudinal direction of a body portion and a distal portion that extends transversely from a distal end of the proximal portion to meet the body portion.
67. The patient interface according to clause 66, wherein a cavity defined between the proximal portion, the distal portion, and the body portion defines a cavity to form one of an expiratory gases receipt opening and an inspiratory gas delivery opening.
68. The patient interface according to clause 67, wherein the other of the inspiratory gas delivery opening and the expiratory gases receipt opening is provided on an opposite side of the distal portion of the flow directing member from the cavity.
69. The patient interface according to any one of clauses 57 to 68, wherein the first and second nasal elements are configured to direct gas into and/or from a nare of the patient.
70. The patient interface according to clause 69, wherein the first nasal element comprises a first nasal prong, and wherein the second nasal element comprises a second nasal prong.
71. The patient interface according to any one of clauses 57 to 70, wherein the cannula manifold is configured to be inserted into the cannula body in the first configuration and in the second configuration.
72. The patient interface according to clause 71, wherein the first configuration corresponds to the cannula manifold being inserted into the cannula body from a first side of the cannula body, and wherein the second configuration corresponds to the cannula manifold being inserted into the cannula body from a second side of the cannula body.
73. The patient interface according to any one of clauses 57 to 72, wherein the cannula body comprises an elastomeric component.
74. The patient interface according to any one of clauses 57 to 73, wherein the cannula manifold comprises a rigid polymer component.
75. The patient interface according to any one of clauses 57 to 74, wherein the cannula manifold comprises a pressure monitoring port, wherein the pressure monitoring port is in fluid communication with the manifold inspiratory lumen and the manifold expiratory lumen.
76. The patient interface according to clause 75, wherein the pressure monitoring port is configured to provide a gas flow for pressure monitoring through the pressure monitoring port, the gas flow comprising an average of the inspiratory gas flow and expiratory gas flow.
77. The patient interface according to any one of clauses 57 to 76, comprising a filter in fluid communication with the manifold expiratory lumen, the filter configured to filter the flow of gas from the patent interface before passage of the flow of expiratory gases from the patient interface into ambient air.
78. A patient interface for respiratory therapy comprising:
   a cannula body comprising a first nasal element configured to direct gas into a nare of the patient and a second nasal element configured to direct gas from another nare of the patient; and
   a cannula manifold, the cannula manifold comprising a manifold inspiratory lumen for inspiratory gas flow in fluid communication with a manifold inlet, a manifold expiratory lumen for expiratory gas flow in fluid communication with a manifold outlet, a flow directing member that is arranged to direct gas flow from the manifold inspiratory lumen to the first nasal element and to direct gas flow from the second nasal element to the manifold expiratory lumen, and a pressure monitoring port in fluid communication with the manifold inspiratory lumen and the manifold expiratory lumen, wherein the manifold inlet and the manifold outlet are on a single limb, such that the manifold inlet and the manifold outlet both extend in one direction from the patient interface in use.
79. The patient interface according to clause 78, comprising a lumen in fluid communication with the pressure monitoring port, an inspiratory gases port in fluid communication with the manifold inspiratory lumen, and an expiratory gases port in fluid communication with the manifold expiratory lumen, wherein the lumen provides fluid communication between the inspiratory gases port, the expiratory gases port, and the pressure monitoring port.
80. The patient interface according to clause 78 or 79, wherein the manifold inlet and the manifold outlet are coaxial or substantially parallel.
81. The patient interface according to any one of clauses 78 to 80, comprising an inspiratory conduit that is in fluid communication with the manifold inspiratory lumen, and an expiratory conduit that is in fluid communication with the manifold expiratory lumen.
82. The patient interface according to clause 81, wherein the inspiratory conduit and the expiratory conduit are integrally formed with the cannula manifold or are coupled to the cannula manifold.
83. The patient interface according to any one of clause 78 to 82, wherein the pressure monitoring port is configured to provide a gas flow for pressure monitoring through the pressure monitoring port, the gas flow comprising an average of the inspiratory gas flow and expiratory gas flow.
84. The patient interface according to any one of clauses 78 to 83, wherein the cannula manifold is configured for insertion into the cannula body.
85. The patient interface according to clause 84, wherein the cannula manifold is configured to be inserted into the cannula body in a first and configuration and in a second configuration, and wherein the first configuration corresponds to the cannula manifold being inserted into the cannula body from a first side of the cannula body, and wherein the second configuration corresponds to the cannula manifold being inserted into the cannula body from a second side of the cannula body.
86. A patient interface for respiratory therapy comprising:
   a cannula body comprising a first nasal element and a second nasal element; and
   a cannula manifold, the cannula manifold comprising a manifold inspiratory lumen, a manifold expiratory lumen, and a flow directing member, wherein the flow directing member is arranged to direct gas flow from the manifold inspiratory lumen to the first nasal element and to direct gas flow from the second nasal element to the manifold expiratory lumen.
87. The patient interface according to clause 86, wherein the cannula manifold is configured for insertion into the cannula body.
88. The patient interface according to clause 86 or 87, comprising or coupled to an inspiratory conduit that is in fluid communication with the inspiratory lumen, and comprising or coupled to an expiratory conduit that in fluid communication with the expiratory lumen.
89. The patient interface according to clause 88, wherein the inspiratory conduit and the expiratory conduit are coaxial or substantially parallel.
90. The patient interface according to clause 89, wherein the inspiratory conduit and the expiratory conduit are on a single limb, such that the inspiratory element and the expiratory element both extend in one direction from the cannula body.
91. The patient interface according to clause 90, wherein the inspiratory conduit is an inner conduit, and wherein the expiratory conduit is an outer conduit.
92. The patient interface according to any one of clauses 86 to 91, wherein the flow directing member is arranged such that a distal one of the first and second nasal elements relative to a manifold inlet and a manifold outlet is an inspiratory nasal element for directing gas flow to the patient and a proximal one of the first and second nasal elements relative to the manifold inlet and the manifold outlet is an expiratory nasal element for directing a flow of expiratory gases away from the patient.
93. The patient interface according to any one of clauses 86 to 91, wherein the flow directing member is arranged such that a proximal one of the first and second nasal elements relative to a manifold inlet and a manifold outlet is an inspiratory nasal element for directing gas flow to the patient and a distal one of the first and second nasal elements relative to the manifold inlet and the manifold outlet is an expiratory nasal element for directing a flow of expiratory gases away from the patient.
94. The patient interface according to clause 92 or 93, comprising a gases port for entrainment of expiratory gases from a patient's nose and/or a patient's mouth that are not directed into the expiratory nasal element.
95. The patient interface according to any one of clauses 86 to 94, wherein the cannula manifold comprises a body portion, and wherein the flow directing member comprises a proximal portion that extends substantially in a longitudinal direction of a body portion and a distal portion that extends transversely from a distal end of the proximal portion to meet the body portion.
96. The patient interface according to clause 95, wherein a cavity defined between the proximal portion, the distal portion, and the body portion defines a cavity to form one of an expiratory gases receipt opening and an inspiratory gas delivery opening.
97. The patient interface according to clause 96, wherein the other of the inspiratory gas delivery opening and the expiratory gases receipt opening is provided on an opposite side of the distal portion of the flow directing member from the cavity.
98. The patient interface according to any one of clauses 96 to 97, wherein the first and second nasal elements are configured to direct gas into and/or from a nare of the patient.
99. The patient interface according to clause 98, wherein the first nasal element comprises a first nasal prong, and wherein the second nasal element comprises a second nasal prong.
100. The patient interface according to any one of clauses 96 to 99, wherein the cannula manifold is configured to be inserted into the cannula body.
101. The patient interface according to any one of clauses 96 to 100, wherein the cannula body comprises an elastomeric component.
102. The patient interface according to any one of clauses 96 to 101, wherein the cannula manifold comprises a rigid polymer component.
103. The patient interface according to any one of clauses 96 to 102, wherein the cannula manifold comprises a pressure monitoring port, wherein the pressure monitoring port is in fluid communication with the manifold inspiratory lumen and the manifold expiratory lumen.
104. The patient interface according to clause 103, wherein the pressure monitoring port is configured to provide a gas flow for pressure monitoring through the pressure monitoring port, the gas flow comprising an average of the inspiratory gas flow and expiratory gas flow.
105. The patient interface according to any one of clauses 96 to 104, comprising a filter in fluid communication with the manifold expiratory lumen, the filter configured to filter the flow of gas from the patent interface before passage of the flow of expiratory gases from the patient interface into ambient air.
106. A respiratory support or assistance system comprising the patient interface according to any one of clauses 37 to 105 and at least one of a humidifier and a respiratory gas source.
107. The respiratory support or assistance system according to clause 106, comprising an inspiratory conduit for delivering gases from the humidifier or respiratory gas source to the patient interface.
108. A kit comprising the tube of any one of clauses 1 to 14 or the expiratory tube of any one of clauses 22 to 32 and a patient interface.
109. The kit according to clause 108, wherein the patient interface is as disclosed in any one of clauses 37 to 105.

## Claims

1. A patient interface for delivery of gas to a patient, the patient interface comprising:
at least one inspiratory lumen for directing a flow of gas to a patient airway;
at least one expiratory lumen comprising an expiratory gas flow path for directing a flow of expiratory gases from the patient; and
a diffuser in fluid communication with, or part of, the expiratory gas flow path, the diffuser configured to diffuse the flow of expiratory gases from the patient interface before passage of the flow of expiratory gases from the patient interface into ambient air.

2. The patient interface according to claim 1, comprising an inspiratory conduit that is in fluid communication with the inspiratory lumen, and an expiratory conduit that is in fluid communication with the expiratory lumen.

3. The patient interface according to claim 2, wherein the inspiratory conduit and the expiratory conduit are integrally formed with the patient interface or are coupled to the patient interface.

4. The patient interface according to claim 2 or 3, wherein the inspiratory conduit and the expiratory conduit are coaxial or substantially parallel.

5. The patient interface according to claim 4, wherein the inspiratory conduit and the expiratory conduit are on a single limb, such that the inspiratory conduit and the expiratory conduit both extend in one direction from the patient interface.

6. The patient interface according to claim 4 or 5, wherein the inspiratory conduit is an inner conduit, and wherein the expiratory conduit is an outer conduit.

7. The patient interface according to any one of claims 1 to 6, wherein a wall of the expiratory conduit comprises the diffuser.

8. The patient interface according to claim 7, wherein a length of the diffuser in the wall is configured to reduce a resistance to flow of gases through the diffuser.

9. The patient interface according to claim 7 or 8, wherein a portion of the expiratory conduit upstream of the diffuser has a wall that is substantially non-permeable.

10. The patient interface according to any one of claims 1 to 9, wherein the expiratory conduit comprises structure to prevent or minimise collapse or constriction of an expiratory lumen in the expiratory conduit, wherein the structure comprises one or more of: corrugations, pleats, helical wrap, ribs, supporting features.

11. The patient interface according to any one of claims 1 to 10, wherein the patient interface comprises first and second nasal elements configured to direct gas into and/or from a nare of the patient, wherein the first nasal element comprises a first nasal prong, and wherein the second nasal element comprises a second nasal prong.

12. The patient interface according to claim11, comprising a flow directing member to direct gas flow to one of the first and second nasal elements, and to direct gas flow from the other of the first and second nasal elements.

13. The patient interface according to claim 11, wherein the patient interface comprises a partition or other physical separation to provide for a mechanical fluid disconnection between a fluid flow path of the first nasal element and a fluid flow path of the second nasal element.

14. The patient interface according to claim 12 or 13, comprising a gases port for entrainment of expiratory gases from a patient's nose and/or a patient's mouth that are not directed into an expiratory nasal element.
